# EUROPEAN PATENT SPECIFICATION

(11) **EP 4 212 509 B1**
(45) Date of publication and mention of the grant of the patent: **27.11.2024**
(21) Application number: 21865739.3
(22) Date of filing: 04.08.2021
(51) Int. Cl.: C07C 201/12, C07C 205/22, C07C 303/30, C07C 309/73, C07C 303/28, C07C 213/02, C07C 215/78, C07C 231/12, C07C 237/04, C07C 269/06, C07C 271/22

(54) **METHOD FOR PREPARING WATER-SOLUBLE MAGNOLOL DERIVATIVE AND HONOKIOL DERIVATIVE, METHODS FOR PREPARING INTERMEDIATES OF WATER-SOLUBLE MAGNOLOL DERIVATIVE AND HONOKIOL DERIVATIVE, AND RELATED MONOHYDROXY PROTECTED INTERMEDIATE**
VERFAHREN ZUR HERSTELLUNG VON WASSERLÖSLICHEM MAGNOLOLDERIVAT UND HONOKIOLDERIVAT, VERFAHREN ZUR HERSTELLUNG VON ZWISCHENPRODUKTEN
PROCÉDÉ DE PRÉPARATION D'UN DÉRIVÉ DE MAGNOLOL HYDROSOLUBLE ET D'UN DÉRIVÉ D'HONOKIOL, PROCÉDÉS DE PRÉPARATION D'INTERMÉDIAIRES DE DÉRIVÉ DE MAGNOLOL HYDROSOLUBLE ET DÉRIVÉ D'HONOKIOL, ET INTERMÉDIAIRE PROTÉGÉ MONOHYDROXY ASSOCIÉ

(30) Priority: 11.09.2020 CN 202010955168
(43) Date of publication of application: 19.07.2023
(73) Proprietor: Beijing Honghui Meditech Co., Ltd, Beijing 102600 (CN)
(72) Inventor: ZHANG, Pingping, Beijing 102600 (CN); LIU, Ye, Beijing 102600 (CN); YU, Guokun, Beijing 102600 (CN); ZHAO, Qiangfeng, Beijing 102600 (CN)
(74) Representative: Casalonga
(86) International application number: PCT/CN2021/110609
(87) International publication number: WO 2022/052683

(56) References cited:
- EP-A1- 2 423 181
- WO-A1-2008/099994
- CN-A- 103 113 264
- CN-A- 103 113 264
- CN-A- 112 125 805
- SOLINAS MAURIZIO ET AL: "Efficient Synthesis of 2-Methyl Derivatives of 1, 1'-Bi(2-naphthol) and 1, 1'-Bi(2-phenols)", EUROPEAN JOURNAL OF ORGANIC CHEMISTRY, vol. 2007, no. 10, 8 March 2007 (2007-03-08), pages 1613 - 1623, XP055910613, ISSN: 1099-0690, DOI: 10.1002/EJOC.200600962
- ZHANG WU, YANG WEN, ZHAO WANXIANG: "Lewis Acid Mediated Electrophilic Cyanation of 2,2′-Biphenols", THE JOURNAL OF ORGANIC CHEMISTRY, AMERICAN CHEMICAL SOCIETY, vol. 85, no. 13, 2 July 2020 (2020-07-02), pages 8702 - 8713, XP055910611, ISSN: 0022-3263, DOI: 10.1021/acs.joc.0c00458
- SOLINAS MAURIZIO, REBECCA E. MEADOWS, CLAIRE WILSON, ALEXANDER J. BLAKE, SIMON WOODWARD: "Efficient Synthesis of 2-Methyl Derivatives of 1, 1'-Bi(2-naphthol) and 1, 1'-Bi(2-phenols)", EUROPEAN JOURNAL OF ORGANIC CHEMISTRY, vol. 2007, no. 10, 8 March 2007 (2007-03-08), pages 1613 - 1623, XP055910613, ISSN: 1099-0690, DOI: 10.1002/EJOC.200600962

## Description

### Technical Field

The present disclosure relates to the technical field of organic synthesis, in particular to a preparation method for water-soluble magnolol derivatives and honokiol derivatives and intermediates thereof, and related monohydroxy protection intermediates.

### Background

Magnolol and honokiol are main active ingredients of traditional Chinese medicine cortex magnoliae officinalis, and the chemical structure formulas of the magnolol and honokiol are as follows:

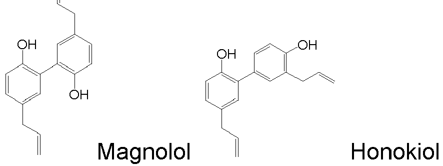

In 1930, the magnolol (Chinese herbal medicine: 2005, 36, 10, 1591-1594) was first isolated from the bark of Chinese magnolia officinalis by Sugii in Japan. In 1989, the honokiol (Chinese patent medicine: 1989, 11 (8): 223.) was also isolated from magnolia officinalis by Meng Lizhen et. al. in China.

The magnolol and honokiol have broad pharmacological effects (Chinese herbal medicine: 2005, 36, 10, 1591-1594), such as antibacterial, anti-inflammatory, anti-tumor, muscle relaxation, cholesterol lowering and anti-aging. However, the water solubility of the magnolol and honokiol is very poor, which seriously limits its wide application in medicine and needs to be improved. Later, the water solubility of the magnolol and honokiol is improved by chemical derivatization. For example, a Chinese patent CN103313264B provides a laboratory preparation method for a magnolol derivative and a honokiol derivative. However, for the preparation method provided in this patent, the selectivity of target products in the synthesis process is relatively poor, the isolation of isomers is difficult, and the multi-step purification needs to be done by column chromatography and the like, it is very difficult to achieve large-scale production.

Based on the above reasons, it is necessary to provide a preparation process for a magnolol derivative and a honokiol derivative, to improve its selectivity and simplify the working procedure, so that it is suitable for the industrial large-scale production.

### Summary

A main purpose of the present disclosure is to provide a preparation method for a water-soluble magnolol derivative and honokiol derivative and an intermediate thereof, and a related monohydroxy protection intermediate, as to solve problems in an existing technology that while the water-soluble magnolol derivative and honokiol derivative are prepared, the selectivity is poor, and the working procedure is complicated, so that the large-scale production may not be performed.

In order to achieve the above purpose, according to one aspect of the present disclosure, a preparation method for a nitrification intermediate of a water-soluble magnolol derivative and honokiol derivative is provided, herein the nitrification intermediate has a structure shown in Formula I:

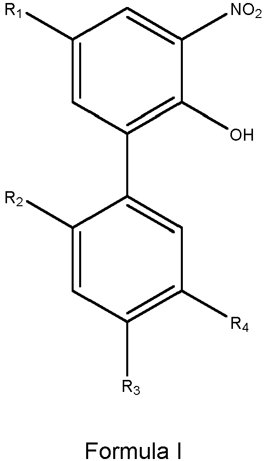

In Formula I, R₂ is a hydroxyl, and R₃ is H; or, R₂ is H, and R₃ is the hydroxyl; and R₁ and R₄ are independently selected from a C₁~C₁₂ electron donor group; and the preparation method includes the following steps: performing monohydroxy protection on a compound A 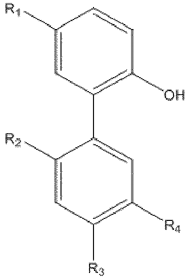 with a hydroxy protection reagent in the presence of an acid binding agent to form a monohydroxy protection compound, herein R₁, R₂, R₃ and R₄ in the compound A have the same definition as above, and the hydroxy protection reagents are p-toluenesulfonyl chloride and 1-hydroxybenzotriazole; and performing a nitrification reaction and a deprotection reaction on the monohydroxy protection compound successively to obtain the nitrification intermediate.

Further, the C₁~C₁₂ electron donor group is selected from a C₁~C₁₂ alkyl or a C₁~C₁₂ alkenyl; preferably, the C₁~C₁₂ electron donor group is selected from methyl, ethyl, n-propyl, isopropyl, n-butyl, isobutyl, tert-butyl, n-pentyl, n-hexyl, vinyl, allyl, octyl, heptyl, propenyl, but-1-enyl, but-2-enyl, but-3-enyl, pent-1-enyl, pent-2-enyl, pent-3-enyl, pent-4-enyl, hex-1-enyl, hex-2-enyl, hex-3-enyl, hex-4-enyl, hex-5-enyl, hept-1-enyl, hept-2-enyl, hept-3-enyl, hept-4-enyl, hept-5-enyl, hept-6-enyl, oct-1-enyl, oct-2-enyl, oct-3-enyl, oct-4-enyl, oct-5-enyl, oct-6-enyl or oct-7-enyl; and preferably, R₁ and R₄ are the same.

Further, in the process of the monohydroxy protection reaction, the molar ratio of the compound A to the p-toluenesulfonyl chloride is 1:(0.75~1), and the molar ratio of the compound A to the 1-hydroxybenzotriazole is 1:(0.75~1); preferably, the monohydroxy protection reaction is performed in a first solvent, and the first solvent is a non-reactive hydrophobic solvent; preferably, the first solvent is selected from one or more of dichloromethane, chloroform, 1,1-dichloroethane, methyl tert-butyl ether and toluene; preferably, the molar ratio of the acid binding agent to the hydroxy protection reagent is (2∼3):1; preferably, the acid binding agent is an organic base; and more preferably, the organic base is selected from one or more of pyridine, 4-dimethylamino-pyridine, 1,8-diazabicycloundeceno-7-ene, triethylamine and N,N-diisopropylethylamine; preferably, the reaction temperature of the monohydroxy protection reaction is -10°C~25°C, more preferably 0~10°C, and the reaction time is 6~10h; and preferably, in the monohydroxy protection reaction, the molar concentration of the compound A is less than 0.2 mol/L relative to the volume of the first solvent, and more preferably, the molar concentration of the compound A is less than 0.1 mol/L.

Further, the step of the nitrification reaction includes: reacting the monohydroxy protection compound with 60∼70wt% nitric acid to obtain a nitrification product; preferably, the nitrification reaction is performed in the second solvent, and the second solvent is a non-reactive solvent; preferably, the second solvent is selected from one or more of dichloromethane, 1,2-dichloroethane, ethyl acetate, methyl tert-butyl ether and acetic acid; preferably, in the process of the nitrification reaction, nitric acid is added dropwise to the second solvent containing the monohydroxy protection compound, and it is reacted at a temperature of 0~25 °C to obtain the nitrification product; and preferably, calculated by 65wt% nitric acid, the weight ratio of the monohydroxy protection compound to the nitric acid is 3.2~4.2:1.

Further, the step of the deprotection reaction includes: mixing the nitrification product with a third solvent, to form mixed solution, herein the third solvent is a non-reactive solvent, and preferably, the third solvent is selected from one or more of 1,4-dioxane, n-propanol, ethylene glycol and toluene; and adding aqueous solution of an alkali metal hydroxide into the mixed solution and heating to react to obtain the nitrification intermediate.

According to another aspect of the present disclosure, a preparation method for an amino substituted intermediate of a water-soluble magnolol derivative and honokiol derivative is provided, herein the amino substituted intermediate has a structure shown in Formula II:

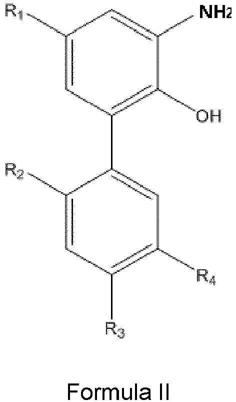

R₁, R₂, R₃ and R₄ in Formula II have the same definition as any one of claims 1 to 5; and the preparation method includes the following steps: using the above preparation method to prepare the nitrification intermediate shown in Formula I; and performing a reduction reaction on the nitrification intermediate, to obtain the amino substituted intermediate.

Further, a reducing reagent used in the step of the reduction reaction includes one of stannous chloride, iron powder, Na₂S and NaHS; preferably, the reduction reaction is performed in a fourth solvent, and the fourth solvent is an alcohol/acid solution system, herein the alcohol is selected from one or more of methanol, ethanol and ethylene glycol, and the acid solution is selected from hydrochloric acid, acetic acid and ammonium chloride aqueous solution; and preferably, in the process of the reduction reaction, a reaction system is heated to reflux.

According to another aspect of the present disclosure, a preparation method for a free base intermediate of a water-soluble magnolol derivative and honokiol derivative is further provided; herein the free base intermediate has a structure shown in Formula III:

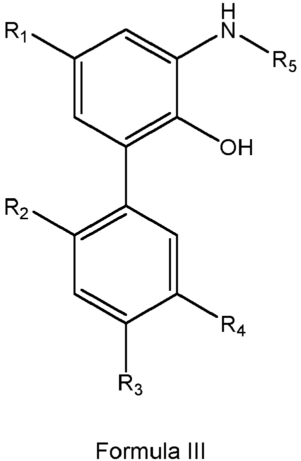

R₁, R₂, R₃ and R₄ in Formula III have the same definition as above, and R₅ is selected from a residue formed by removing a hydroxyl of a carboxyl of a single amino acid or a peptide during a condensation reaction of the carboxyl; and the preparation method includes the following steps: using the above preparation method to prepare the amino substituted intermediate shown in Formula II; performing the condensation reaction between the amino substituted intermediate and a single amino acid protected by tert-butoxycarbonyl or a peptide protected by tert-butoxycarbonyl, to obtain a condensation product; and reacting the condensation product with hydrogen chloride, and then alkalizing by ammonia water, extracting and recrystallizing to obtain the free base intermediate.

Further, the single amino acid is selected from one of lysine, methionine, tryptophan, valine, alanine, phenylalanine, leucine, isoleucine, glycine, histidine, arginine, proline, glutamate, cystine and aspartic acid, and the molecular weight of the peptide is ≤2500 Da; preferably, the molar ratio of the amino substituent intermediate to the single amino acid protected by tert-butoxycarbonyl or the peptide protected by tert-butoxycarbonyl is (1.2~0.8):1; preferably, the condensation reaction is performed in a fifth solvent, the fifth solvent is a non-reactive solvent, and preferably, the fifth solvent is selected from one or more of dichloromethane, 1,2-dichloroethane, ethyl acetate, tetrahydrofuran, and N,N-dimethylformamide; preferably, in the process of the condensation reaction, the reaction temperature is 0~30 °C; preferably, the reaction process between the condensation product and hydrogen chloride is performed in a sixth solvent, the sixth solvent is a non-reactive solvent, and preferably, the sixth solvent is selected from one or more of ether, ethyl acetate, dichloromethane and 1,4-dioxane.

According to another aspect of the present disclosure, a preparation method for a water-soluble magnolol derivative and honokiol derivative is further provided, herein the water-soluble magnolol derivative and honokiol derivative have a structure shown in Formula IV:

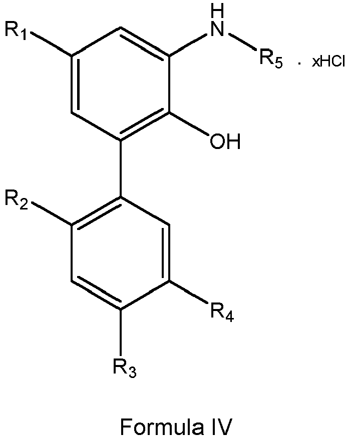

R₁, R₂, R₃, R₄ and R₅ in Formula IV have the same definition as above; x is the number of salified amino groups contained in R₅; and the preparation method includes the following steps: using the above preparation method to prepare the free base intermediate shown in Formula III; reacting the free base intermediate with a hydrochloric acid, concentrating or freeze-drying to obtain the water-soluble magnolol derivative and honokiol derivative.

According to another aspect of the present disclosure, a monohydroxy protection intermediate of a water-soluble magnolol derivative and honokiol derivative is further provided, herein the monohydroxy protection intermediate has a structure shown in Formula V below:

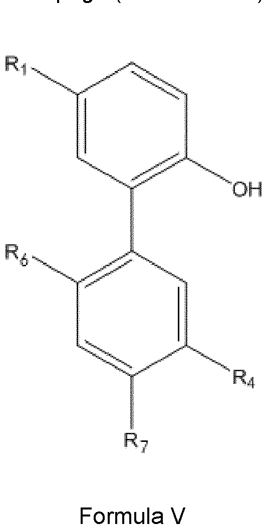

Herein, in Formula V, R₁ and R₄ have the same definition as above; R₆ is 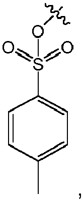 and R₇ is H; or, R₆ is H, and R₇ is

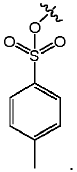

The present disclosure provides a preparation method for a nitrification intermediate of a water-soluble magnolol derivative and honokiol derivative, it uses the active ester formed by the p-toluenesulfonyl chloride and the 1-hydroxybenzotriazole under the existence of the acid binding agent as the hydroxy protection reagent, and after the monohydroxy of the compound A is protected, the nitrification reaction and the deprotection reaction are performed successively to obtain the nitrification intermediate

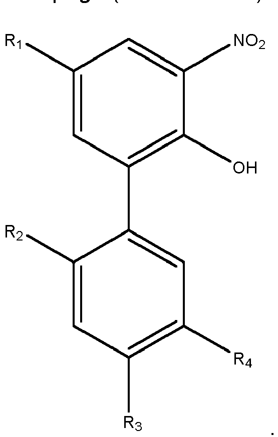

The compound A has a parent nucleus structure of the magnolol or honokiol, and its two benzene rings have the hydroxyl respectively. The present disclosure uses the p-toluenesulfonyl chloride and 1-hydroxybenzotriazole as the hydroxy protection reagent, and the active ester formed by it may achieve selective single protection of the hydroxyl represented by R₂ and R₃ in the double hydroxyls of the magnolol derivative and honokiol derivative, thereby the selectivity in the subsequent nitrification reaction process is effectively improved, namely the nitrification reaction may occur in an ortho-position of the unprotected hydroxyl. After the nitrification reaction is completed, the nitrification intermediate with the structure of Formula I may be obtained by the deprotection reaction. The nitrification intermediate is used for subsequent nitro reduction, condensation reaction with the amino acid or peptide protected by tert-butoxycarbonyl, tert-butoxycarbonyl deprotection reaction and other reactions to obtain the water-soluble magnolol derivative and honokiol derivative.

In a word, the use of the preparation method provided by the present disclosure effectively improves the selectivity of the nitrification reaction of the compound A, and correspondingly improves the synthesis efficiency of the water-soluble magnolol derivative and honokiol derivative; and on the one hand, it is beneficial to improve the yield of the target product, and on the other hand, it may completely eliminate the column chromatography steps, significantly simplify the synthesis working procedure, reduce the production difficulty, and meet the requirements of the industrial large-scale production.

### Detailed Description of the Embodiments

It should be noted that embodiments of the present application and features in the embodiments may be combined with each other in the case without conflicting. The present disclosure is described in detail below in combination with the embodiments.

As described in the background, in the existing technology, while the water-soluble magnolol derivative and honokiol derivative are prepared, there are the problems that the selectivity is low, and the working procedure is complicated, so that the large-scale production may not be performed.

In order to solve the above problems, the present disclosure provides a preparation method for a nitrification intermediate of a water-soluble magnolol derivative and honokiol derivative, and the nitrification intermediate has a structure shown in Formula I:

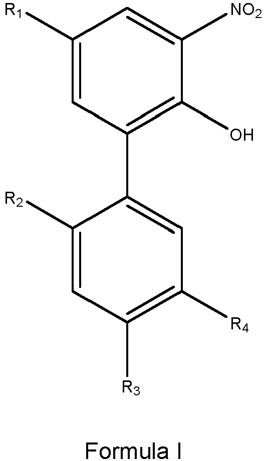

In Formula I, R₂ is a hydroxyl, and R₃ is H; or, R₂ is H, and R₃ is the hydroxyl; and R₁ and R₄ are independently selected from a C₁~C₁₂ electron donor group; and the preparation method includes the following steps: performing monohydroxy protection on a compound A 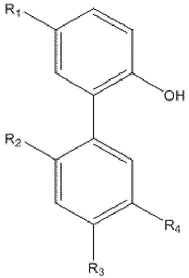 with a hydroxy protection reagent in the presence of an acid binding agent to form a monohydroxy protection compound, herein R₁, R₂, R₃ and R₄ in the compound A have the same definition as above, and the hydroxy protection reagents are p-toluenesulfonyl chloride and 1-hydroxybenzotriazole; and performing a nitrification reaction and a deprotection reaction on the monohydroxy protection compound successively to obtain the nitrification intermediate.

The compound A has a parent nucleus structure of the magnolol or honokiol, and its two benzene rings have the hydroxyl respectively. The present disclosure uses an active ester formed by the p-toluenesulfonyl chloride and 1-hydroxybenzotriazole under the existence of the acid binding agent as the hydroxy protection reagent, and the active ester may achieve the selective single protection of the hydroxyl represented by R₂ and R₃ in the double hydroxyls of the magnolol derivative and honokiol derivative, thereby the selectivity in the subsequent nitrification reaction process is effectively improved, namely the nitrification reaction may occur in an ortho-position of the unprotected hydroxyl. After the nitrification reaction is completed, the nitrification intermediate with the structure of Formula I may be obtained by the deprotection reaction. The honokiol compound A is taken as an example, and the specific reaction route is as follows:

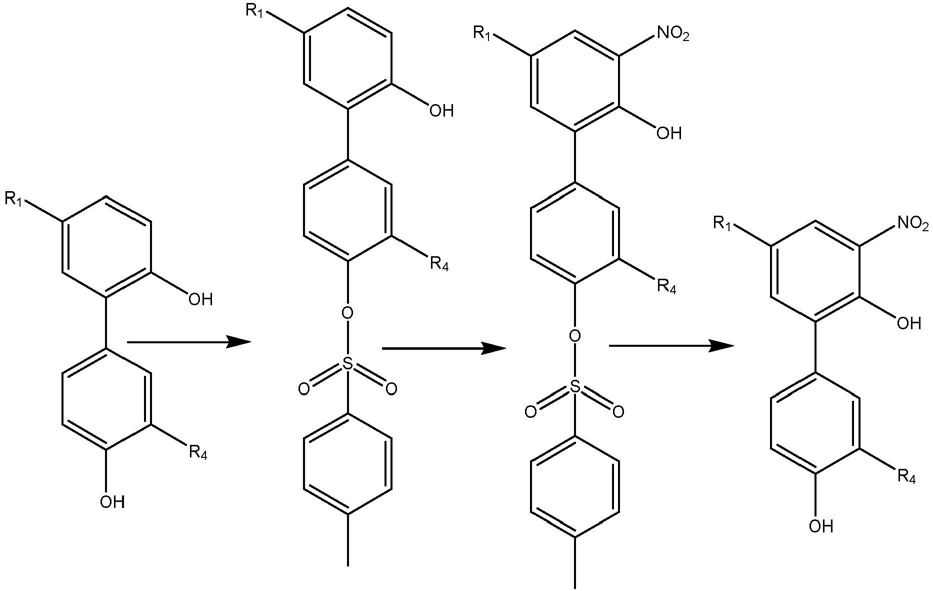

The nitrification intermediate is used for subsequent nitro reduction, condensation reaction with the amino acid protected by the tert-butoxycarbonyl, tert-butoxycarbonyl deprotection, acid and alkali adjustment and other reactions, to obtain the water-soluble magnolol derivative and honokiol derivative.

It should be noted that the active ester formed by the p-toluenesulfonyl chloride and 1-hydroxybenzotriazole is less active than a direct esterification reaction with toluenesulfonyl chloride, and the steric structure of the active ester is larger than that of the toluenesulfonyl chloride, which may further reduce the reaction chance of the hydroxyl with greater steric hindrance. The above reasons enable the compound A with the magnolol and honokiol type double hydroxyl structure to obtain the selective monohydroxy protection.

In addition, for a protection reagent with the larger steric hindrance, such as octanyl chloride and lauroyl chloride, it is found by the inventor that the hydroxyl position selectively protected by it is exactly opposite to the protected position required by the above compound A. This shows that the activity of the two hydroxyls in the compound A is high or low (especially for the compound A with the honokiol parent nucleus structure). The high or low activity of the two hydroxyls is opposite to the size of the steric hindrance. Although the molecular size of the octanoyl chloride and the lauroyl chloride is relatively large, due to the flexible structure of a fatty chain, the difference in the activity of the hydroxyls plays a leading role in the reaction. On the contrary, the active ester formed by the hydroxy protection agent in the present disclosure is a rigid structure of an aromatic ring, so that steric hindrance plays a leading role in the reaction. Based on these researches, the present disclosure achieves the technical schemes of the selective monohydroxy protection of the compound A by using the active ester formed by the p-toluenesulfonyl chloride and 1-hydroxybenzotriazole as the hydroxy protection reagent, thereby the synthesis selectivity of the whole water-soluble magnolol derivative and honokiol derivative is effectively improved, the difficulty of purification and separation is reduced, and the working procedure is simplified, so that it may be industrially applied on a large scale.

In addition, in the compound A of the present disclosure, R₂ is a hydroxyl, and R₃ is H; or, R₂ is H, and R₃ is the hydroxyl; R₁ and R₄ are independently selected from a C₁~C₁₂ electron donor group respectively. While R₂ is the hydroxyl and R₃ is H, the nitrification intermediate of the magnolol derivative is prepared; and while R₂ is H and R₃ is the hydroxyl, the nitrification intermediate of the honokiol derivatives is prepared. R₁ and R₄ are independently selected from the C₁~C₁₂ electron donor group respectively, and the use of the electron donating effect of these groups may further change the selectivity of the hydroxyls in R₂ and R₃ positions in the process of the monohydroxy protection reaction. In a word, the use of the preparation method provided by the present disclosure effectively improves the selectivity of the nitrification reaction of the compound A, and correspondingly improves the synthesis efficiency of the water-soluble magnolol derivative and honokiol derivative; and on the one hand, it is beneficial to improve the yield of the target product, and on the other hand, it may also reduce the difficulty of product purification and separation at each reaction stage, significantly simplify the synthesis working procedure, reduce the production difficulty, and meet the requirements of the industrial large-scale production.

In order to further improve the selectivity of the monohydroxy protection and nitrification reaction, in a preferred implementation mode, the C₁~C₁₂ electron donor group is selected from a C₁~C₁₂ alkyl or a C₁~C₁₂ alkenyl; preferably, the C₁~C₁₂ electron donor group is selected from methyl, ethyl, n-propyl, isopropyl, n-butyl, isobutyl, tert-butyl, n-pentyl, n-hexyl, vinyl, allyl, octyl, heptyl, propenyl, but-1-enyl, but-2-enyl, but-3-enyl, pent-1-enyl, pent-2-enyl, pent-3-enyl, pent-4-enyl, hex-1-enyl, hex-2-enyl, hex-3-enyl, hex-4-enyl, hex-5-enyl, hept-1-enyl, hept-2-enyl, hept-3-enyl, hept-4-enyl, hept-5-enyl, hept-6-enyl, oct-1-enyl, oct-2-enyl, oct-3-enyl, oct-4-enyl, oct-5-enyl, oct-6-enyl or oct-7-enyl; and more preferably, the above R₁ and R₄ are the same.

More preferably, R₁ and R₄ are independently selected from a C₁~C₈ electron donor group respectively, such as a C₁~C₈ alkyl or a C₁~C₈ alkenyl.

In a preferred implementation mode, in the process of the monohydroxy protection reaction, the molar ratio of the compound A to the p-toluenesulfonyl chloride is 1:(0.75-1), and the molar ratio of the compound A to the 1-hydroxybenzotriazole is 1:(0.75-1); and preferably, the acid binding agent is an organic base, and its molar ratio to the p-toluenesulfonyl chloride is (2~3):1. The amount relationship between raw materials is controlled within the above range, it is more beneficial to perform the monohydroxy protection reaction, thereby the reaction efficiency is further improved.

For the purpose of further improving the reaction stability and promoting the efficient reaction, in a preferred implementation mode, the monohydroxy protection reaction is performed in a first solvent, and the first solvent is a non-reactive hydrophobic solvent. In the actual reaction process, p-toluenesulfonic chloride, 1-hydroxybenzotriazole and an acid binding agent are usually first added in the first solvent to react to form the active ester, and then the compound A is added to further react to obtain a monohydroxy protection intermediate compound. More preferably, the reaction temperature is controlled at 0-25 °C, and further preferably, the reaction temperature is in the range of 0~10°C, and the reaction time is 6~10 h. Preferably, the organic base is selected from pyridine, 4-dimethylamino-pyridine, 1,8-diazabicycloundeceno-7-ene, triethylamine and N,N-diisopropylethylamine; and the first solvent is selected from one or more of dichloromethane, chloroform, 1,1-dichloroethane, methyl tert-butyl ether and toluene. In this way, the reaction selectivity is better, the efficiency is higher, and the reaction stability and safety is higher.

In order to further improve the selectivity and efficiency of the nitrification reaction, in a preferred implementation mode, the step of the nitrification reaction includes: reacting the monohydroxy protection compound with 60∼70wt% nitric acid, to obtain a nitrification product. Preferably, the nitrification reaction is performed in a second non-reactive solvent, and preferably the second solvent is selected from one or more of dichloromethane, 1,2-dichloroethane, ethyl acetate, methyl tert-butyl ether and acetic acid. It is performed in the above solvent, the nitrification reaction is more stable, and there are fewer by-products. Preferably, in the process of the nitrification reaction, the nitric acid is added dropwise to the second solvent containing the monohydroxy protection compound, and it is reacted at a temperature of 0~25 °C, to obtain the nitrification product; and preferably, the weight ratio of the monohydroxy protection compound to the nitric acid is 3.2∼4.2:1 (calculated by 65wt% of the nitric acid). The nitration reaction is safer and more stable under the above reaction conditions and raw material amount ratio conditions.

In a preferred implementation mode, the step of the deprotection reaction includes: mixing the nitrification product with a third solvent, to form mixed solution, herein the third solvent is a non-reactive solvent, and the third solvent is selected from one or more of 1,4-dioxane, n-propanol, ethylene glycol and toluene; and adding aqueous solution of an alkali metal hydroxide into the mixed solution, and reacting, to obtain the nitrification intermediate.

According to another aspect of the present disclosure, a preparation method for an amino substituted intermediate of a water-soluble magnolol derivative and honokiol derivative is further provided, herein the amino substituted intermediate has a structure shown in Formula II:

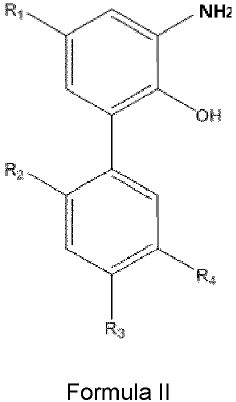

R₁, R₂, R₃ and R₄ in Formula II have the same definition as above; and the preparation method includes the following steps: using the above preparation method to prepare the nitrification intermediate shown in Formula I; and performing a reduction reaction on the nitrification intermediate, to obtain the amino substituted intermediate.

In the actual operation process, in order to improve the reduction reaction efficiency, in a preferred implementation mode, a reducing reagent used in the step of the reduction reaction includes one of stannous chloride, iron powder, Na₂S and NaHS; preferably, the reduction reaction is performed in a fourth solvent, and the fourth solvent is an alcohol/acid solution mixed solvent, herein the alcohol is selected from one or more of methanol, ethanol and ethylene glycol, and the acid solution is selected from hydrochloric acid, acetic acid and ammonium chloride aqueous solution; and preferably, in the process of the reduction reaction, a reaction system is heated to a reflux state.

According to another aspect of the present disclosure, a preparation method for a free base intermediate of a water-soluble magnolol derivative and honokiol derivative is provided, herein the free base intermediate has a structure shown in Formula III:

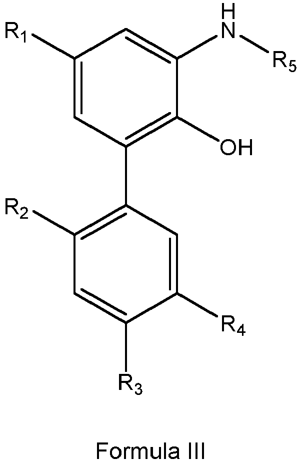

R₁, R₂, R₃ and R₄ in Formula III have the same definition as above, and R₅ is selected from a residue formed by removing a hydroxyl of a carboxyl of a single amino acid or a peptide during a condensation reaction of the carboxyl; and the preparation method includes the following steps: using the above preparation method to prepare the amino substituted intermediate shown in Formula II; performing the condensation reaction between the amino substituted intermediate and a single amino acid protected by tert-butoxycarbonyl or a peptide protected by tert-butoxycarbonyl, to obtain a condensation product; and reacting the condensation product with hydrogen chloride, and then alkalizing by ammonia water, extracting and recrystallizing to obtain the free base intermediate.

In a preferred implementation mode, the single amino acid is selected from one of lysine, methionine, tryptophan, valine, alanine, phenylalanine, leucine, isoleucine, glycine, histidine, arginine, proline, glutamate, cystine and aspartic acid, and the molecular weight of the peptide is ≤2500 Da (preferably formed from the above amino acids). In order to further improve the reaction efficiency and improve the yield of the target compound, in a preferred implementation mode, the molar ratio of the amino substituent intermediate to the amino acid protected by the tert-butoxycarbonyl or the peptide protected by the tert-butoxycarbonyl is (1.2~0.8):1; preferably, the condensation reaction is performed in a fifth solvent, the fifth solvent is selected from one or more of dichloromethane, 1,2-dichloroethane, ethyl acetate, tetrahydrofuran, and N,N-dimethylformamide; preferably, in the process of the condensation reaction, the reaction temperature is 0~30 °C; preferably, the reaction process between the condensation product and the hydrogen chloride is performed in a sixth solvent, and the sixth solvent is selected from one or more of ether, ethyl acetate, dichloromethane and 1,4-dioxane.

According to another aspect of the present disclosure, a preparation method for a water-soluble magnolol derivative and honokiol derivative is provided, herein the water-soluble magnolol derivative and honokiol derivative have a structure shown in Formula IV:

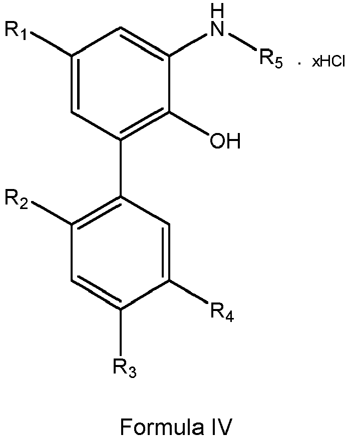

R₁, R₂, R₃, R₄ and R₅ in Formula IV have the same definition as above; x is the number of salified amino groups contained in R₅; and the preparation method includes the following steps: preparing the free base intermediate shown in Formula III, and reacting the free base intermediate with a hydrochloric acid, to obtain the water-soluble magnolol derivative and honokiol derivative.

In the actual operation process, the free base intermediate may be acidified with the hydrochloric acid and freeze-dried to obtain the water-soluble magnolol derivative and honokiol derivative shown in Formula IV. The concentration of the hydrochloric acid may be any concentrations, for example, a commercially available hydrochloric acid with a concentration of 37wt% may be used, and more preferably, a diluted hydrochloric acid with a concentration of 1∼10wt% is used.

Based on the higher selectivity of the previously described monohydroxy protection reaction and nitrification reaction in the present disclosure, the deprotection reaction using alkali metal hydroxide, the condensation reaction, the deprotection reaction using hydrogen chloride, the alkalization operation using ammonia water, extraction, crystallization, acidification and freeze-drying are all simple and easy to operate, so that the preparation of the water-soluble magnolol derivative and honokiol derivative shown in Formula IV has the better conversion rate and yield, and it is easy to be produced industrially.

According to another aspect of the present disclosure, a monohydroxy protection intermediate of a water-soluble magnolol derivative and honokiol derivative is further provided, and it has a structure shown in Formula V below:

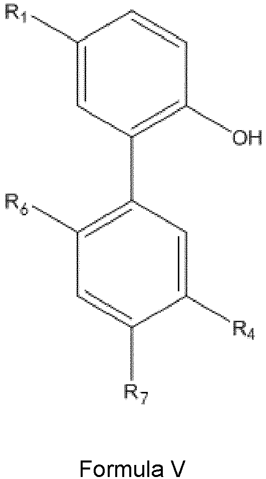

Herein, in Formula V, R₁ and R₄ have the same definition as above; R₆ is 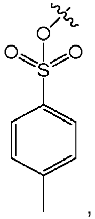 and R₇ is H; or, R₆ is H, and R₇ is

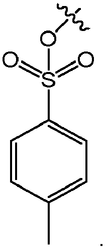

The above monohydroxy protection compound is prepared by the following preparation method: performing monohydroxy protection on the above compound A 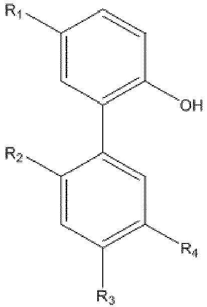 with a hydroxy protection reagent in the presence of the acid binding agent, to form a monohydroxy protection compound, herein R₁, R₂, R₃ and R₄ in the compound A have the same definition as above, and the hydroxy protection reagent is p-toluenesulfonyl chloride and 1-hydroxybenzotriazole.

The present application is further described in detail below in combination with specific embodiments, and these embodiments may not be understood as limiting a scope of protection claimed by the present application.

Abbreviated terms involved in the embodiments: DCC: 1,3-dicyclohexylcarbodiimide; TsCI: p-toluenesulfonyl chloride; HOBT: 1-hydroxybenzotriazole; DIEA: N,N-diisopropylethylamine; SnCl₂: stannous chloride; and 1,4-dioxane.

### Embodiment 1

A nitrification intermediate (compound 6) of a honokiol derivative was prepared by the following reaction route

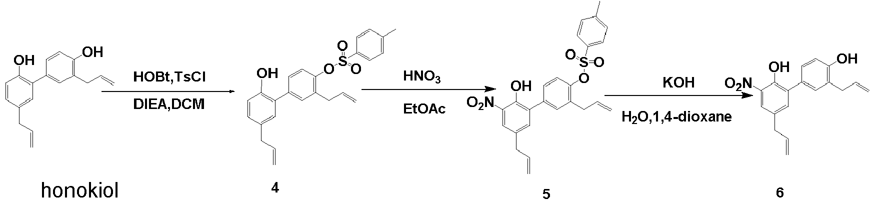

### Step I:

### Preparation of 3, 5'-diallyl-2'-hydroxy-[1,1'-biphenyl]-4-yl 4-methylbenzenesulfonate (compound 4)

Dichloromethane (160 kg) was added to a 200 L reaction kettle, it was stirred and the internal temperature was cooled within 10°C. HOBT (0.744 kg), DIEA (1.61 kg) and TsCI (0.954 kg) was added in sequence. After 30 min, TLC showed that TsCI disappeared. Honokiol (1.6 kg) was added, the internal temperature was controlled at 5±5°C, and it was continuously stirred for 6 h. 1 N aq. HCl (20 L) was added slowly, and the mixture was stirred for 30 min, stood and layered. The organic layer was washed with 1N aq. HCl (20 L) again. Then the organic layer was added into the reaction kettle, 1 N aq.NaOH (20 L) was added, and the mixture was stirred, stood and layered. The organic layer was washed with 1N aq. NaOH (20 L) again. After that, the organic layer was added into the reaction kettle, brine (20 L) was added, and the mixture was stirred, stood and layered. The organic layer was dried over anhydrous Na₂SO₄, filtered and concentrated. Isopropanol (1.91 L) was added into the residue, it was heated, stirred and dissolved. After being cooled, a solid was precipitated and filtered out. The mother liquid was concentrated under a reduced pressure to obtain compound 4 (1.28 kg, 87% purity) as a yellow oily with a yield of 61 %.

Compound 4: C₂₅H₂₄O₄S=420.14, MS:438[M+NH₄]⁺. ¹H-NMR(300MHz, CDCl₃) δ9.46(1H,S), 7.83-7.81(2H,d), 7.52-7.49(2H,d), 7.39-7.37(2H,d), 7.00-6.97(3H,m), 6.85-6.82(1H,m) 5,98-5,69(2H,m), 5.06-4.96(4H,m), 3.34-3.17(4H,m), 2.48-2.35(3H,m).

### Step II:

### Preparation of 3,5'-diallyl-2'-hydroxy-3'-nitro-[1,1'-biphenyl]-4-yl 4-methylbenzenesulfonate (compound 5)

Dichloromethane (5 L) was added to a 10 L reaction kettle with stirring, compound 4 (1.28 kg) was added, and the mixture was cooled to 0 °C. 65%wt aq. HNO₃ (0.304 kg) was dropwise added within 30min and then the reaction mixture was stirred for additional 2h. TLC showed that the reaction was completed. Pure water (2.6 L) was added and the mixture was stirred for 15 min, stood and layered. The organic layer was washed with sat.aq.NaHCOs (2.5 L) and brine (2.5 L) successively, dried over anhydrous Na₂SO₄, filtered, and concentrated under the reduced pressure. Methanol (8 L) was added into the residue, it was heated, stirred and dissolved, and then cooled to 0 °C. It was filtered, and the filter cake was dried to obtain compound 5 (863.5g, 97% purity) as a yellow solid with a yield of 61%.

Compound 5: C₂₅H₂₃NO₆S₂=465.52, MS: 483[M+NH₄]⁺. ¹H-NMR(400MHz, CDCl₃) δ11.00(1H,s), 7.93(1H,d)7.82(2H,d), 7.43-7.32(5H,m), 7.12(2H,d), 5.98-5.75(2H,m), 5.16-5.02(4H,m), 3.40-3.29(4H,dd), 2.47(3H,s).

### Step III:

### Preparation of 3',5-diallyl-3-nitro-[1,1'-biphenyl]-2,4'-diol (compound 6)

1,4-dioxane (5 L) and the compound 5 (0.862 kg) were added into a 10 L reaction kettle, and the mixture was stirred and dissolved. KOH (0.521 kg) was dissolved in water (1.7 L), it was added dropwise within 20 min and the reaction mixture was stirred at 85 °C for 4h. TLC showed that the reaction was completed. The mixture was cooled, and concentrated under the reduced pressure. Dichloromethane (5 L) and water (2.5 L) were added into the residue, and conc.aq.HCl was added dropwise with stirring until PH=5, and the mixture was stood and layered. The organic phase was washed with sat.aq. NaHCOs (2.5 L) and brine (2.5 L) successively, dried with the anhydrous Na₂SO₄, filtered, and concentrated under the reduced pressure. Dichloromethane (1 L) was added and the residue was dissolved with heating. N-hexane (8 L) was added and the resulting mixture was stirred at 0 °C for 1h and then filtered. The filter cake was dried to obtain compound 3 (0.425 kg, 95% purity) as a dark red solid with a yield of 74%.

Compound 6: C₁₈H₁₇NO₄=311.33, MS: 329[M+NH₄]⁺. ¹H-NMR(400MHz, CDCl₃) δ10.03(1H, s), 7.90(1H, d), 7.35-7.31(2H, m), 6.90(1H, d), 6.11-5.90(2H, m), 5.24-5.11(5H,m), 3.48(2H,d), 3.40(2H, d).

### Embodiment 2

An amino substituted intermediate (compound 7) of a honokiol derivative was prepared by the following reaction route

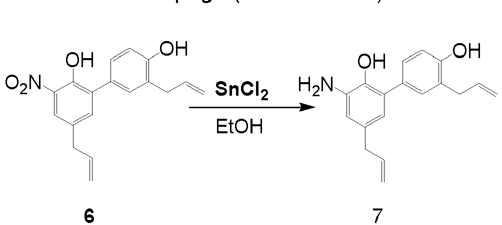

### Preparation of 3', 5-diallyl-3-amino-[1,1'-biphenyl]-2,4'-diol (compound 7)

To a 10 L reaction kettle was added ethanol (2 L) and compound 6 (0.248 kg) with stirring, and then stannous chloride (0.629 kg) was added. The resulting reaction mixture was refluxed for 2h. TLC showed that the reaction was completed. It was cooled to room temperature and concentrated under a reduced pressure. Ethyl acetate (5L) was added and stirred. Sat.aq.NaHCO₃ was added dropwise slowly until pH=8 and a large amount of a solid was formed. The mixture was filtered and the filtrate was stood and layered. The organic phase was collected, a filter cake was washed with ethyl acetate (5 L x 3), and the combined organic phase was concentrated under the reduced pressure to obtain compound 7 (0.179 kg, 96% purity) as an earthy yellow solid with a yield of 80%.

Compound 7: C₁₈H₁₉NO₂= 281.14, MS:282[M+H]⁺, ¹H-NMR(400MHz, DMSO) δ9.925(1H, s), 9.59(1H, s), 9.22 (1H, s)9.22, 7.12(1H, t), 7.06(1H, d), 7.03(1H, d), 5.99-5.86(2H, m), 5.10-4.98(4H, m), 3.32(4H, dd).

### Embodiment 3

A hydrochloride compound (compound 10) of a honokiol derivative was prepared by the following reaction route

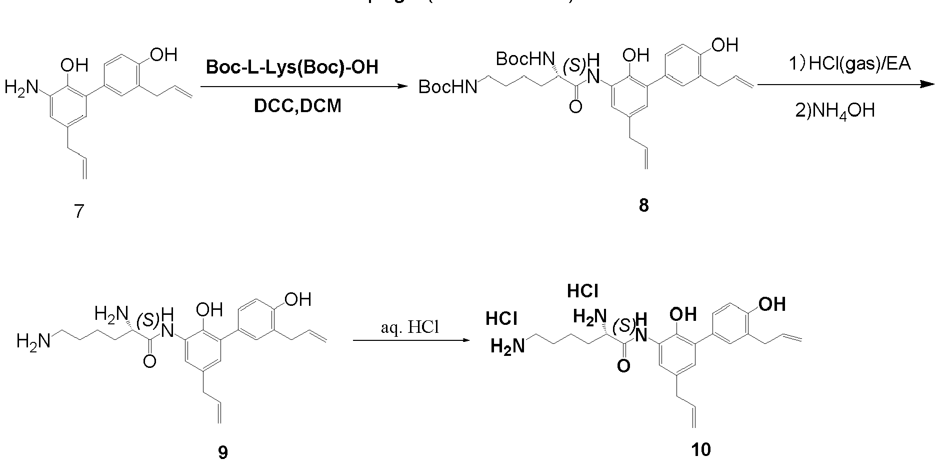

### Step I:

### Preparation of di-tert- butyl (6-((3', 5-diallyl-2,4'-dihydroxy-[1,1'-biphenyl]-3-yl)amino)-6-oxohexane-1,5-diyl)(S)-dicarbamate (compound 8)

Dichloromethane (2.5 L), DCC (103.3 g) and Boc-L-lys (Boc)-OH (172.3 g) were successively added into a 5 L reaction flask, and the mixture was stirred at 0 °C. After 30min, compound 7 (139.5 g) was added and then the reaction mixture was stirred for 2h. It was filtered and the filtrate was concentrated to obtain compound 8 (324 g, 87% purity) as a gray foam-like solid.

Compound 8: C₃₄H₄₇N₃O₇=609.75, MS: 610[M+H]+. ¹H NMR(300MHz, CDCl₃) δ8.637(1H, s), 7.316(5H, m), 6.892(2H, m), 6.099(3H, m), 5.357(1H, s), 5.206(4H, m), 4.670(1H, s), 4.288(1H, t), 3.445(2H, d), 3.314(2H, d), 3.133(2H, t), 2.056(2H, m), 1.460(22H, m).

### Step II:

### Preparation of (S)-2,6-diamino-N-(3',5-diallyl-2,4'-dihydroxy-[1,1'-biphenyl]-3-yl)hexanamide (compound 9)

A 5 L reaction kettle was replaced with nitrogen gas for three times, and 4.0 M hydrogen chloride/ethyl acetate solution (1.5 L) was added, stirred and cooled to 0 °C. Compound 8 (321.1g) was added slowly, the reaction mixture was stirred at 0 °C for 5h and a solid was generated. TLC showed that compound 8 disappeared. Water (1L) was added, the organic phase was isolated and the aqueous phase was adjusted to pH=9 with ammonia water. A solid was formed and it was extracted with ethyl acetate (1 L x 2). The combined organic phase was evaporated and the residue was crystallized twice in a mixed solution of ethyl acetate and n-hexane to obtain compound 10 (96.8 g, 98% purity, and 48% yield for two-steps) as a yellow solid.

Compound 9: C₂₄H₃₁N₃O₃=409.53, MS: 410. 410[M+H]⁺, ¹H -NMR (400MHz, MeOH) δ7.52(1H, d), δ7.24(2H, m), δ6.84(2H, t), δ6.08(2H, m), 5.11-4.94(4H, m), 3.41(1H, t), 3.39(2H,d), 3.32(2H,d), 2.66(2H,t), 1.83(1H,m), 1.66(1H,m), 1.53(4H,m).

### Step III:

### Preparation of (S)-2,6-diamino-N-(3',5-diallyl-2,4'-dihydroxy-[1,1'-biphenyl]-3-yl)hexanamide dihydrochloride (compound 10)

Compound 9 (41 g) was dissolved in 1 M dilute aq. HCl (210 ml) under stirring at 0 °C, and the solution was freeze-dried to obtain compound 10 (48.2 g, 97.8% purity, and 100% yield) as a yellowish solid.

Compound 10: C₂₄H₃₃Cl₂N₃O₃=482.44, MS:410[M+H]⁺(free state). ¹H -NMR (400MHz, DMSO) δ10.43(1H, s), δ9.46(1H, s), δ8.61(1H, s), δ8.43(3H, s), 7.99(3H,s), 7.33(1H,s), 7.16(2H,t), 6.86(2H,d), 5.99(2H,m), 5.09(4H,m), 4.15(1H,t), 3.29(4H,m), 2.77(2H,t), 1.89(2H,q), 1.63(2H,m), 1.47(2H,m).

### Embodiment 4

A nitrification intermediate (compound 13) of a magnolol derivative was prepared by the following reaction route:

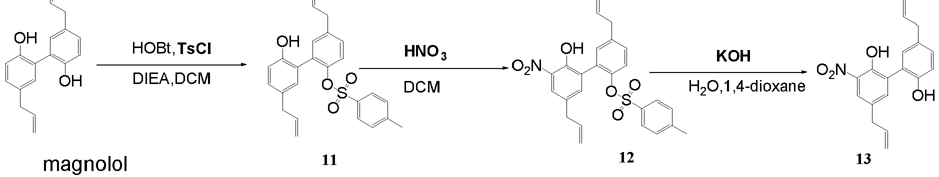

### Step I:

### Preparation of 5,5'-diallyl-2'-hydroxy-[1,1'-biphenyl]-2-yl 4-methylbenzenesulfonate (compound 11)

Dichloromethane (3 L) was added to a 5 L reaction flask, it was stirred and the internal temperature was cooled within 10 °C. HOBT (18.5 g), DIEA (40.1 g) and TsCI (23.7 g) was added in sequence. After 30min, TLC showed that TsCI disappeared. Magnolol (40.0 g) was added and the reaction mixture was continuously stirred at 5±5°C for 6 h. 1 N aq.HCl (500 mL) was added slowly. After being stirred for 30 min, it was stood and layered. The organic layer was washed with 1 N aq.HCl (500 mL) once more. 1 N aq NaOH (500 mL) was added, and it was stirred, stood and layered. The organic layer was washed with 1 N aq.NaOH (500 mL) once more. Brine (500 mL) was added. After being stirred for 30min, it was stood and layered. The organic phase was dried over anhydrous Na₂SO₄, filtered and concentrated. Isopropanol (50 mL) was added to the residue, it was heated, stirred and dissolved. After being cooled, a precipitate was formed and filtered out. The mother liquid was concentrated under a reduced pressure to obtain compound 11 (33 kg, 87% purity) as a yellow oily with a yield of 57.3%.

Compound 11: C₂₅H₂₄O₄S=420.14, MS:438 [M+NH₄]⁺.

### Step II:

### Preparation of 5,5'-diallyl-2'-hydroxy-3'-nitro-[1,1'-biphenyl]-2-yl 4-methylbenzenesulfonate (compound 12)

To a 250 mL reaction kettle with stirring was added dichloromethane (120 mL) and compound 11 (30.1 g) and the mixture was cooled to 0 °C. 65%wt aq HNO₃ (7.15 g) was dropwise added within 30min and the reaction mixture was continuously stirred for 2h. TLC showed that the reaction was completed. Water (60 mL) was added. After being stirred for 15min, it was stood and layered. The organic phase was washed with sat.aq.NaHCOs (60 mL) and brine (60 mL) successively, dried over anhydrous Na₂SO₄, filtered and concentrated under the reduced pressure. Methanol (8 L) was added into the residue, it was heated, stirred and dissolved, and then cooled to 0 °C. The mixture was filtered and the filter cake is dried to obtain compound 12(20.1 g, 98% purity) as a yellow solid with a yield of 60%.

### Step III:

### Preparation of 5,5'-diallyl-3-nitro-[1,1'-biphenyl]-2,2'-diol (compound 13)

To a 250 mL reaction flask was added 1,4-dioxane (125 mL) and compound 12 (20 g) and the mixture was stirred and dissolved. Potassium hydroxide (2.8 g) was dissolved in water (50 mL), it was dropwise added within 20 min, and then it was reacted at 85 °C for 4 h. TLC showed that the reaction was completed. After being cooled, the mixture was concentrated under the reduced pressure. Dichloromethane (250 mL) and water (120 mL) were added into the residue, conc.aq.HCl was dropwise added under stirring until PH=5 and it was stood and layered. The organic phase was washed with sat.aq.NaHCOs (120 mL) and brine (120 mL) successively, dried over anhydrous Na₂SO₄, filtered and concentrated under the reduced pressure to obtain compound 13 (13 g, 97% purity) as a dark red solid with a yield of 97%.

Compound 13: C₁₈H₁₇NO₄=311.12, MS: 329[M+NH₄]⁺, ¹H-NMR(300MHz , CDCl₃) δ8.01(1H,d), 7.50(1H, d), 7.18(1H, dd), 7.01(1H, dd), 6.91(1H, dd), 6.04-5.88(2H, m), 5.18-5.06(4H, m), 3.42(4H, dd).

### Embodiment 5

### Influence of reaction temperature on selective ratio of 3,5'-diallyl-2'-hydroxy-[1,1'-biphenyl]-4-yl 4-methylbenzenesulfonate (compound 4) to 3,5'-diallyl-4'-hydroxy-[1,1'-biphenyl]-2-yl 4-methylbenzenesulfonate (compound 4a)

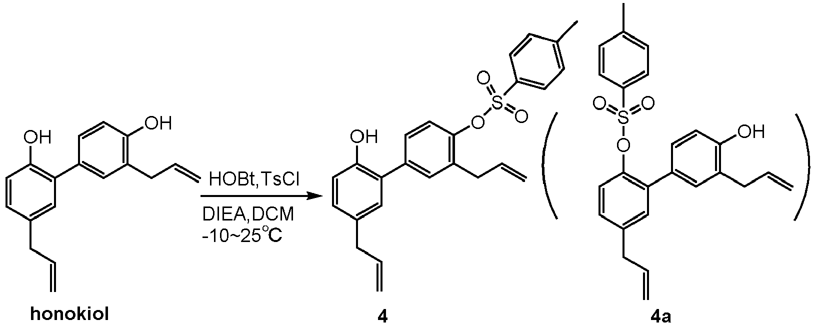

Method: Dichloromethane (1 L) was added into a stirred 2 L three necked flask and cooled to -10 °C (other three batches were cooled to 0 °C, 10 °C and 25 °C respectively), HOBT (12.4 g) DIEA (26.9 g) and TsCI (15.9 g) were added in sequence After 30min. Honokiol (26.6 g) was added to the reaction flask, and the reaction mixture was continuously stirred for 6 h. LC-MS monitored a ratio of compound 4 to compound 4a.

Experimental results were shown in the following table:

| Number | Temperature | Compound 4:4a ratio |
|---|---|---|
| 1 | -10 °C | 14:1 |
| 2 | 0 °C | 14:1 |
| 3 | 10 °C | 14:1 |
| 4 | 25 °C | 10:1 |

Conclusion: In the range of -10 °C to 25 °C, the ratios of compound 4 to 4a are all greater than 10:1, and the selectivity is good. In the range of -10 °C to 10 °C, the reaction selectivity is as high as 14:1. The selectivity at 25 °C is slightly poor. The reaction selectivity, devices and energy conservation and the like are further comprehensively considered, the temperature range is preferably 0 °C -10 °C.

### Embodiment 6

### Influence of reactant concentration on selective ratio of 3,5'-diallyl-2'-hydroxy-[1,1'-biphenyl]-4-yl 4-methylbenzenesulfonate (compound 4) to 3,5'-diallyl-4'-hydroxy-[1,1'-biphenyl]-2-yl 4-methylbenzenesulfonate (compound 4a)

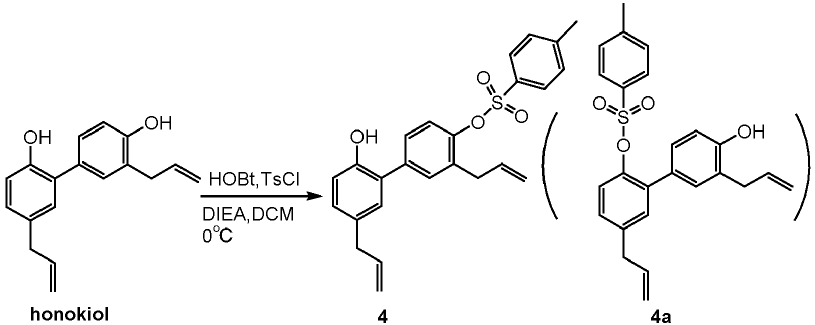

Method: 1) 500 mL of dichloromethane (other two batches are 1 L and 2 L respectively) was added into a stirred 2 L three necked flask and cooled to 0 °C, HOBT (12.4 g) DIEA (26.9 g) and TsCI (15.9 g) were added in sequence. After 30 min, honokiol (26.6g) was added and the mixture was continuously stirred for 6 h. LC-MS monitored a ratio of compound 4 to compound 4a.

Experimental results were shown in the following table:

| Number | Concentration | Compound 4:4a ratio |
|---|---|---|
| 1 | 0.2mol/L(26.6g/500ml) | 10:1 |
| 2 | 0.1mol/L(26.6g/1L) | 12:1 |
| 3 | 0.05mol/L(26.6g/2L) | 15:1 |

Conclusion: while the reactant concentration is less than 0.2 mol/L, the ratios of compound 4 to 4a are all greater than 10:1, and the selectivity is good; and further preferably, the concentration is less than 0.1mol/L.

### Embodiment 7:

The compound 7 is used as a starting material, the following compounds 14-17 may also be obtained according to the preparation method of the present disclosure.

| Compound name | Compound structure | Data |
|---|---|---|
| (S)-2-amino-N-(3',5-di allyl-2,4'-dihydroxy-[1, 1'-biphenyl]-3-yl)-3-ph enylpropanamide hydrochloride (compound 14) | 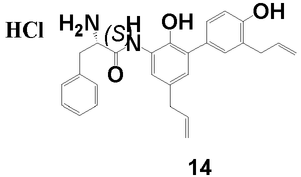 | C₂₇H₂₉ClN₂O₃=464.99, |
| | | MS 429.1[M+H]⁺(free base). |
| | | ¹H-NMR(400MHz, CDCl₃) δ10.29(1H,s),9.46(1H,s), 8.51(1H, s), 8.42(3H,t), 7.31-7.29(5H,m), 7.18-7.14(2H,m),7.09-7.08 (1H,d)6.87-6.85(2H,t)6.00-5.88(2 H,m), 5.11-4.99(4H,m), 4.40-4.37(2H,t),3.33-3.10(6H, m). |
| (R)-2,6-diamino-N-(3', 5-diallyl-2,4'-dihydroxy -[1,1'-biphenyl]-3-yl)he xanamide | 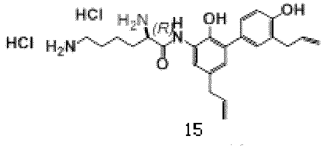 | C₂₄H₃₃Cl₂N₃O₃=482.44, MS:410[M+H]⁺(free base). |
| | | ¹H -NMR (400MHz, DMSO) δ10.43(1H, s), δ9.46(1H, s), δ8.61(1H, s), δ8.43(3H,s), 7.99(3H,s), 7.36(1H,s), 7.18-7.14(2H,t), 6.87-6.85(2H,d), 6.00-5.90(2H,m), 5.11-4.99(4H,m), 4.16(1H,d), 3.29(4H,m), 2.78(2H,t), 1.89(2H,q), 1.64(2H,m), 1.48(2H,m). |
| .dihydrochloride (compound 15) | | |
| (S)-2-amino-N-(3',5-di allyl-2,4'-dihydroxy-[1, 1'-biphenyl]-3-yl)-3-m ethylbutanamide hydrochloride (compound 16) | 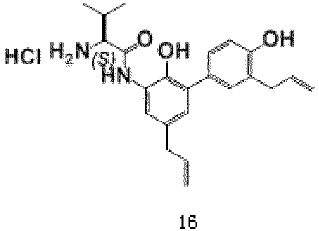 | C₂₃H₂₉ClN₂O₃=416.95, |
| | | MS: 381.2[M+H]⁺(free base). |
| | | ¹H -NMR (400MHz, DMSO) δ10.32(1H, s), δ9.45(1H, s), δ8.56(1H, s), δ8.34(3H, s), 7.34(1H,s), 7.19-7.14(2H,m), 6.87-6.85(2H,q), 6.01-5.90(2H,m), 5.11-5.00(4H,m), 4.00(1H,t), 3.33-3.29(4H,m), 2.23(1H,m), 1.17(6H,d). |
| (S)-2-amino-N-(3',5-di allyl-2,4'-dihydroxy-[1, 1'-biphenyl]-3-yl)-6-hy droxyhexanamide hydrochloride (compound 17) | 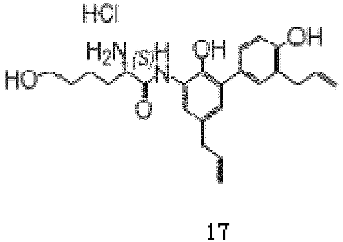 | C₂₃H₂₉ClN₂O₃S=449.01, |
| | | MS: 413.3[M+H]⁺(free base). |
| | | ¹H -NMR (400MHz, DMSO) δ10.19(1H, s), δ9.45(1H, s), δ8.55(1H, s), δ8.36(3H, s), 7.34(1H,s), 7.19-7.14(2H,m), 6.86-6.84(2H,t), 6.00-5.90(2H,m), 5.11-5.00(4H,m), 4.19(1H,t), 3.39-3.29(4H,m), 2.61-2.57(2H,m), 1.84(2H,q), 2.14-2.09(5H,m). |

### Contrast example 1

A nitrification intermediate (compound 3) of a honokiol derivative was prepared by the following reaction route

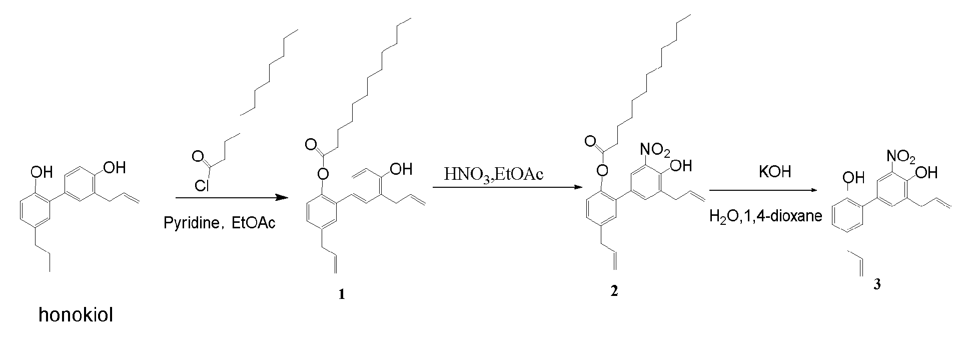

### Step I:

### Preparation of 3',5-diallyl-4'-hydroxy-[1,1'-biphenyl]-2-yl dodecanoate (compound 1)

Honokiol (5 g, 1 eq) was dissolved in an ethyl acetate (50 ml) and the solution was stirred at 0°C. Pyridine (3.9 g, 2 eq) was added, and lauroyl chloride (4.5 g, 1.1 eq) was dropwise added. After that, the reaction mixture was stirred overnight. It was diluted with ethyl acetate (50 ml) washed successively with 1 N aq. HCl, sat.aq.NaHCOsand brine. The organic phase was dried over anhydrous Na₂SO₄, filtered and concentrated. The crude product was purified by column chromatography (ethyl acetate: petroleum ether=1:20) to obtain compound 1 (3.6 g, 95% purity) with a yield of 43%.

Compound 1: C₃₀H₄₀O₃=448.30, MS: 446[M+NH₄]⁺. ¹H-NMR(300MHz, CDCl₃) δ7.19(4H,m),7.03(1H,d),6.81(1H,d),6.01(2H,m),5.18(4H,m),3.42(4H,m)2.36(2H,t),1.66(2H,m), 1.25(16H,m),0.89(3H,t).

### Step II:

### Preparation of 3', 5-diallyl-4'-hydroxy-5'-nitro-[1,1'-biphenyl]-2-yl dodecanoate (compound 2)

Compound 1 (2 g, 4.5 mmol) was dissolved in acetic anhydride (5 ml) and the solution was stirred at 0°C. 65%wt aq.HNO₃ (0.8 ml) was dissolved in acetic acid (6 ml) and it was dropwise added. After being added, the reaction mixture was continuously stirred at 0 °C for 1h. It was poured into ice water and extracted with ethyl acetate (50 ml x2).The organic phase was dried over anhydrous Na₂SO₄, filtered and concentrated to afford a crude product, which was purified by column chromatography (petroleum ether: ethyl acetate=30:1) to obtain compound 2 (410 mg) with yield of 18%.

Compound 2: C₃₀H₃₉NO₅=493.28, MS: 511[M+NH₄]⁺.

### Step III:

### Preparation of 3', 5-diallyl-5'-nitro-[1,1'-biphenyl]-2,4'-diol (compound 3)

Compound 2 (300mg, 0.6mmol) was dissolved in 1,4-dioxane(5 ml) and water (5 ml), NaOH (100 mg, 2.5mmol) was added, and the mixture was stirred at a room temperature for 2h. It was adjusted to be acidic with 1 N aq.HCl and extracted with ethyl acetate. The organic phase was washed successively with sat.aq.NaHCO₃ and brine, dried over anhydrous Na₂SO₄, filtered and concentrated. The crude product was purified by column chromatography (petroleum ether: ethyl acetate=15:1) to obtain compound 3 (140 mg, 95% purity) with a yield of 70%.

Compound 3: C₁₈H₁₇NO₄=311.12, MS: 329[M+NH₄]⁺. ¹H NMR(300MHz, DMSO) δ10.385(1H, s), 9.579(3H, s), 7.707(1H,s), 7.422(1H,s), 7.200(2H,m), 6.869(1H,s), 5.974(2H,m), 5.062(4H,m), 3.398(4H,m).

It may be seen from the above data that the above embodiments of the present disclosure achieve the following technical effects.

By adopting the process provided by the present disclosure, the reaction selectivity is effectively improved, and the working procedure is simpler. In the pilot-scale production process of the embodiment, the selectivity and yield are still higher, and the synthesis efficiency is higher.

The above are only preferred embodiments of the present disclosure, and are not intended to limit the present disclosure. For those skilled in the art, the present disclosure may have various changes and variations.

## Claims

1. A preparation method for a nitrification intermediate of a water-soluble magnolol derivative and honokiol derivative, wherein the nitrification intermediate has a structure shown in Formula I: 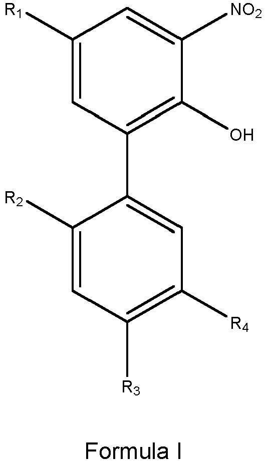
in Formula I, R₂ is a hydroxyl, and R₃ is H; or, R₂ is H, and R₃ is a hydroxyl; and R₁ and R₄ are independently selected from C₁~C₁₂ electron donor groups; and
the preparation method comprises the following steps:
performing monohydroxy protection on a compound A 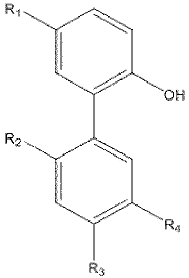 with a hydroxyl protection reagent in the presence of an acid binding agent to form a monohydroxy protection compound, wherein R₁, R₂, R₃ and R₄ in the compound A have the same definition as above, and the hydroxy protection reagents are p-toluenesulfonyl chloride and 1-hydroxybenzotriazole; and
performing a nitrification reaction and a deprotection reaction on the monohydroxy protection compound successively to obtain the nitrification intermediate.

2. The preparation method for the nitrification intermediate of the water-soluble magnolol derivative and honokiol derivative according to claim 1, wherein the C₁~C₁₂ electron donor group is selected from C₁~C₁₂ alkyl or C₁~C₁₂ alkenyl;
preferably, the C₁~C₁₂ electron donor group is selected from methyl, ethyl, n-propyl, isopropyl, n-butyl, isobutyl, tert-butyl, n-pentyl, n-hexyl, vinyl, allyl, octyl, heptyl, propenyl, but-1-enyl, but-2-enyl, but-3-enyl, pent-1-enyl, pent-2-enyl, pent-3-enyl, pent-4-enyl, hex-1-enyl, hex-2-enyl, hex-3-enyl, hex-4-enyl, hex-5-enyl, hept-1-enyl, hept-2-enyl, hept-3-enyl, hept-4-enyl, hept-5-enyl, hept-6-enyl, oct-1-enyl, oct-2-enyl, oct-3-enyl, oct-4-enyl, oct-5-enyl, oct-6-enyl or oct-7-enyl; and
preferably, R₁ and R₄ are the same.

3. The preparation method for the nitrification intermediate of the water-soluble magnolol derivative and honokiol derivative according to claim 1, wherein in the process of the monohydroxy protection reaction, the molar ratio of the compound A to the p-toluenesulfonyl chloride is 1:(0.75~1), and the molar ratio of the compound A to the 1-hydroxybenzotriazole is 1:(0.75-1);
preferably, the monohydroxy protection reaction is performed in a first solvent, and the first solvent is a non-reactive hydrophobic solvent;
preferably, the first solvent is selected from one or more of dichloromethane, chloroform, 1,1-dichloroethane, methyl tert-butyl ether and toluene;
preferably, the molar ratio of the acid binding agent to the hydroxy protection reagent is (2~3):1;
preferably, the acid binding agent is an organic base; and more preferably, the organic base is selected from one or more of pyridine, 4-dimethylamino-pyridine, 1,8-diazabicycloundeceno-7-ene, triethylamine and N,N-diisopropylethylamine;
preferably, the reaction temperature of the monohydroxy protection reaction is -10 °C~25 °C, more preferably 0~10 °C, and the reaction time is 6~10 h; and
preferably, in the monohydroxy protection reaction, the molar concentration of the compound A is less than 0.2 mol/L relative to the volume of the first solvent, and more preferably, the molar concentration of the compound A is less than 0.1 mol/L.

4. The preparation method for the nitrification intermediate of the water-soluble magnolol derivative and honokiol derivative according to any one of claims 1 to 3, wherein the step of the nitrification reaction comprises: reacting the monohydroxy protection compound with 60∼70wt% nitric acid to obtain a nitrification product;
preferably, the nitrification reaction is performed in a second solvent, and the second solvent is a non-reactive solvent;
preferably, the second solvent is selected from one or more of dichloromethane, 1,2-dichloroethane, ethyl acetate, methyl tert-butyl ether and acetic acid;
preferably, in the process of the nitrification reaction, the nitric acid is added to the second solvent containing the monohydroxy protection compound in a dropwise adding mode, and it is reacted at a temperature range of 0-25 °C to obtain the nitrification product; and
preferably, calculated by 65wt% nitric acid, the weight ratio of the monohydroxy protection compound to nitric acid is 3.2∼4.2:1.

5. The preparation method for the nitrification intermediate of the water-soluble magnolol derivative and honokiol derivative according to claim 1, wherein the step of the deprotection reaction comprises:
mixing the nitrification product with a third solvent to form a mixed solution, wherein the third solvent is a non-reactive solvent, and preferably, the third solvent is selected from one or more of 1,4-dioxane, n-propanol, ethylene glycol and toluene; and
adding aqueous solution of an alkali metal hydroxide into the mixed solution and heating, and reacting to obtain the nitrification intermediate.

6. A preparation method for an amino substituted intermediate of a water-soluble magnolol derivative and honokiol derivative, wherein the amino substituted intermediate has a structure shown in Formula II: 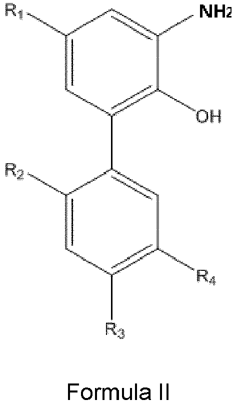 R₁, R₂, R₃ and R₄ in Formula II have the same definition as any one of claims 1 to 5; and the preparation method comprises the following steps:
using the preparation method according to any one of claims 1 to 5 to prepare the nitrification intermediate shown in Formula I; and
performing a reduction reaction on the nitrification intermediate to obtain the amino substituted intermediate.

7. The preparation method for the amino substituted intermediate of the water-soluble magnolol derivative and honokiol derivative according to claim 6, wherein a reducing reagent used in the step of the reduction reaction comprises one of stannous chloride, iron powder, Na₂S and NaHS;
preferably, the reduction reaction is performed in a fourth solvent, and the fourth solvent is an alcohol/acid mixed solvent, wherein the alcohol is selected from one or more of methanol, ethanol and ethylene glycol, and the acid is selected from hydrochloric acid, acetic acid and ammonium chloride aqueous solution; and
preferably, in the process of the reduction reaction, a reaction system is heated to reflux.

8. A preparation method for a free base intermediate of a water-soluble magnolol derivative and honokiol derivative, wherein the free base intermediate has a structure shown in Formula III: 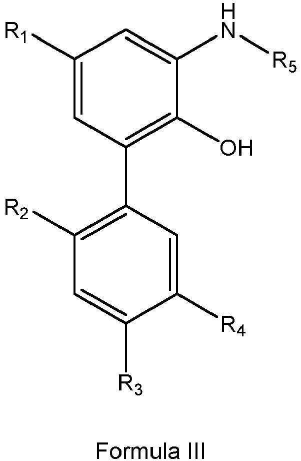
R₁, R₂, R₃ and R₄ in Formula III have the same definition as any one of claims 1 to 5, and R₅ is selected from a acyl residue formed by removing a hydroxyl of a carboxyl of a single amino acid or a peptide during a condensation reaction of the carboxyl; and
the preparation method comprises the following steps:
using the preparation method according to claim 6 or 7 to prepare the amino substituted intermediate shown in Formula II;
performing the condensation reaction between the amino substituted intermediate and a single amino acid protected by tert-butoxycarbonyl or a peptide protected by the tert-butoxycarbonyl to obtain a condensation product; and
reacting the condensation product with hydrogen chloride, and then alkalizing by ammonia water, extracting and recrystallizing to obtain the free base intermediate.

9. The preparation method for the free base intermediate of the water-soluble magnolol derivative and honokiol derivative according to claim 8, wherein the single amino acid is selected from one of lysine, methionine, tryptophan, valine, alanine, phenylalanine, leucine, isoleucine, glycine, histidine, arginine, proline, glutamate, cystine and aspartic acid, and the molecular weight of the peptide is ≤2500 Da;
preferably, the molar ratio of the amino substituent intermediate to the single amino acid protected by tert-butoxycarbonyl or the peptide protected by tert-butoxycarbonyl is (1.2~0.8):1;
preferably, the condensation reaction is performed in a fifth solvent, the fifth solvent is a non-reactive solvent, and preferably, the fifth solvent is selected from one or more of dichloromethane, 1,2-dichloroethane, ethyl acetate, tetrahydrofuran, and N,N-dimethylformamide;
preferably, in the process of the condensation reaction, the reaction temperature is 0~30°C;
preferably, the reaction process between the condensation product and hydrogen chloride is performed in a sixth solvent, the sixth solvent is a non-reactive solvent, and preferably, the sixth solvent is selected from one or more of ether, ethyl acetate, dichloromethane and 1,4-dioxane.

10. A preparation method for a water-soluble magnolol derivative and honokiol derivative, wherein the water-soluble magnolol derivative and honokiol derivative have a structure shown in Formula IV: 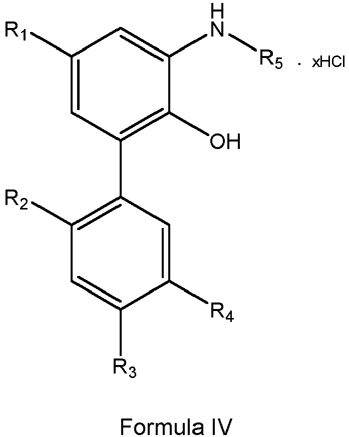 R₁, R₂, R₃, R₄ and R₅ in Formula IV have the same definition as that in claim 8 or 9; x is the number of salified amino groups contained in R₅; and the preparation method comprises the following steps:
using the preparation method according to claim 8 or 9 to prepare the free base intermediate shown in Formula III;
reacting the free base intermediate with hydrochloric acid, concentrating or freeze-drying, to obtain the water-soluble magnolol derivative and honokiol derivative.

11. A monohydroxy protection intermediate of a water-soluble magnolol derivative and honokiol derivative, wherein the monohydroxy protection intermediate has a structure shown in Formula V below: 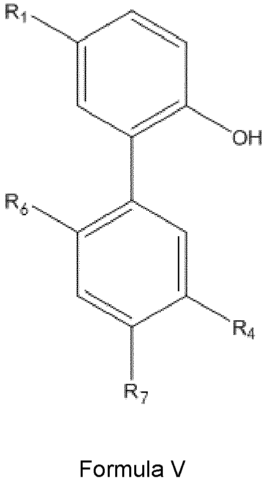 wherein, in Formula V, R₁ and R₄ have the same definition as above; R₆ is 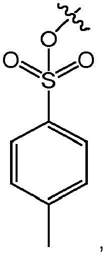 and R₇ is H; or, R₆ is H, and R₇ is

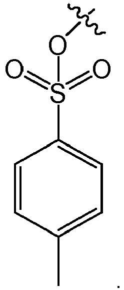

## Patentansprüche

1. Herstellungsverfahren für ein Nitrifizierungszwischenprodukt eines wasserlöslichen Magnololderivats und Honokiolderivats, wobei das Nitrifizierungszwischenprodukt eine in Formel I dargestellte Struktur hat: 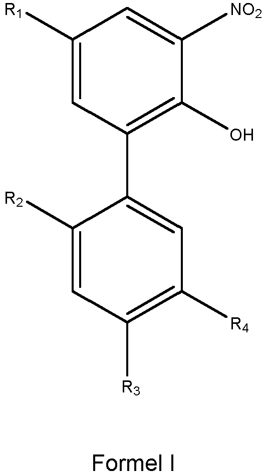
in Formel I ist R₂ ein Hydroxyl und R₃ ist H; oder R₂ ist H und R₃ ist ein Hydroxyl; und R₁ und R₄ sind unabhängig voneinander ausgewählt aus C₁~C₁₂-Elektronendonatorgruppen; und
das Herstellungsverfahren umfasst die folgenden Schritte:
Durchführen von Monohydroxyschutz an einer Verbindung A 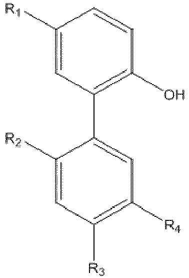 mit einem Hydroxylschutzreagens in Gegenwart eines Säurebindungsmittels, um eine Monohydroxyschutzverbindung zu bilden, wobei R₁, R₂, R₃ und R₄ in Verbindung A dieselbe Definition haben wie oben und die Hydroxyschutzreagenzien p-Toluolsulfonylchlorid und 1-Hydroxybenzotriazol sind; und
Durchführen einer Nitrifizierungsreaktion und einer Entschützungsreaktion nacheinander an der Monohydroxyschutzverbindung, um das Nitrifizierungszwischenprodukt zu erhalten.

2. Herstellungsverfahren für das Nitrifizierungszwischenprodukt des wasserlöslichen Magnololderivats und Honokiolderivats nach Anspruch 1, wobei die C₁~C₁₂-Elektronendonatorgruppe aus C₁~C₁₂-Alkyl oder C₁~C₁₂-Alkenyl ausgewählt ist;
vorzugsweise ist die C₁~C₁₂-Elektronendonatorgruppe ausgewählt aus Methyl, Ethyl, n-Propyl, Isopropyl, n-Butyl, Isobutyl, tert-Butyl, n-Pentyl, n-Hexyl, Vinyl, Allyl, Octyl, Heptyl, Propenyl, But-1-enyl, But-2-enyl, But-3-enyl, Pent-1-enyl, Pent-2-enyl, Pent-3-enyl, Pent-4-enyl, Hex-1-enyl, Hex-2-enyl, Hex-3-enyl, Hex-4-enyl, Hex-5-enyl, Hept-1-enyl, Hept-2-enyl, Hept-3-enyl, Hept-4-enyl, Hept-5-enyl, Hept-6-enyl, Oct-1-enyl, Oct-2-enyl, Oct-3-enyl, Oct-4-enyl, Oct-5-enyl, Oct-6-enyl oder Oct-7-enyl; und
vorzugsweise sind R₁ und R₄ identisch.

3. Herstellungsverfahren für das Nitrifizierungszwischenprodukt des wasserlöslichen Magnololderivats und Honokiolderivats nach Anspruch 1, wobei bei dem Prozess der Monohydroxyschutzreaktion das Molverhältnis der Verbindung A zu dem p-Toluolsulfonylchlorid 1:(0,75~1) beträgt und das Molverhältnis der Verbindung A zu dem 1-Hydroxybenzotriazol 1:(0,75~1) beträgt;
vorzugsweise wird die Monohydroxyschutzreaktion in einem ersten Lösungsmittel durchgeführt, und das erste Lösungsmittel ist ein nichtreaktives hydrophobes Lösungsmittel;
vorzugsweise ist das erste Lösungsmittel ausgewählt aus einem oder mehreren von Dichlormethan, Chloroform, 1,1-Dichlorethan, Methyl-tert-butylether und Toluol;
vorzugsweise beträgt das Molverhältnis des Säurebindungsmittels zu dem Hydroxyschutzreagens (2~3):1;
vorzugsweise ist das Säurebindungsmittel eine organische Base; und mehr bevorzugt ist die organische Base ausgewählt aus einem oder mehreren von Pyridin, 4-Dimethylamino-pyridin; 1,8-Diazabicycloundeceno-7-en, Triethylamin und N,N-Diisopropylethylamin;
vorzugsweise beträgt die Reaktionstemperatur der Monohydroxyschutzreaktion -10°C~25°C, mehr bevorzugt 0-10°C, und die Reaktionszeit beträgt 6-10 h; und
vorzugsweise beträgt die molare Konzentration der Verbindung A bei der Monohydroxyschutzreaktion weniger als 0,2 mol/L bezogen auf das Volumen des ersten Lösungsmittels, und mehr bevorzugt beträgt die molare Konzentration der Verbindung A weniger als 0,1 mol/L.

4. Herstellungsverfahren für das Nitrifizierungszwischenprodukt des wasserlöslichen Magnololderivats und Honokiolderivats nach einem der Ansprüche 1 bis 3, wobei der Schritt der Nitrifizierungsreaktion Folgendes umfasst: Umsetzen der Monohydroxyschutzverbindung mit 60-70 Gew.-% Salpetersäure, um ein Nitrifizierungsprodukt zu erhalten;
vorzugsweise wird die Nitrifizierungsreaktion in einem zweiten Lösungsmittel durchgeführt, und das zweite Lösungsmittel ist ein nichtreaktives Lösungsmittel;
vorzugsweise ist das zweite Lösungsmittel ausgewählt aus einem oder mehreren von Dichlormethan, 1,2-Dichlorethan, Ethylacetat, Methyl-tert-butylether und Essigsäure;
vorzugsweise wird die Salpetersäure bei dem Prozess der Nitrifizierungsreaktion dem die Monohydroxyschutzverbindung enthaltenden zweiten Lösungsmittel in einem Modus der tropfenweisen Zugabe zugesetzt, und sie wird in einem Temperaturbereich von 0~25°C umgesetzt, um das Nitrifizierungsprodukt zu erhalten; und
vorzugsweise, berechnet mit 65 Gew.-% Salpetersäure, beträgt das Gewichtsverhältnis der Monohydroxyschutzverbindung zu Salpetersäure 3,2-4,2:1.

5. Herstellungsverfahren für das Nitrifizierungszwischenprodukt des wasserlöslichen Magnololderivats und Honokiolderivats nach Anspruch 1, wobei der Schritt der Entschützungsreaktion Folgendes umfasst:
Mischen des Nitrifizierungsprodukts mit einem dritten Lösungsmittel, um eine gemischte Lösung zu bilden, wobei das dritte Lösungsmittel ein nichtreaktives Lösungsmittel ist, und vorzugsweise ist das dritte Lösungsmittel ausgewählt aus einem oder mehreren von 1,4-Dioxan, n-Propanol, Ethylenglykol und Toluol; und
Zugabe von wässriger Lösung eines Alkalimetallhydroxids in die gemischte Lösung und Erhitzen, sowie Umsetzen, um das Nitrifizierungszwischenprodukt zu erhalten.

6. Herstellungsverfahren für ein aminosubstituiertes Zwischenprodukt eines wasserlöslichen Magnololderivats und Honokiolderivats, wobei das aminosubstituierte Zwischenprodukt eine in Formel II dargestellte Struktur hat: 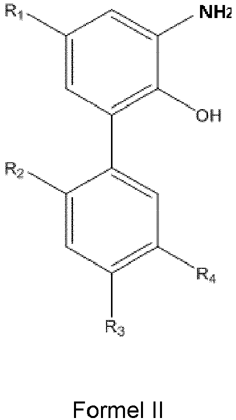
R₁, R₂, R₃ und R₄ in Formel II haben dieselbe Definition wie in einem der Ansprüche 1 bis 5; und
das Herstellungsverfahren umfasst die folgenden Schritte:
Verwenden des Herstellungsverfahrens nach einem der Ansprüche 1 bis 5, um das in Formel I dargestellte Nitrifizierungszwischenprodukt herzustellen; und
Durchführen einer Reduktionsreaktion an dem Nitrifizierungszwischenprodukt, um das aminosubstituierte Zwischenprodukt zu erhalten.

7. Herstellungsverfahren für das aminosubstituierte Zwischenprodukt des wasserlöslichen Magnololderivats und Honokiolderivats nach Anspruch 6, wobei ein bei dem Schritt der Reduktionsreaktion verwendetes Reduktionsmittel eines von Zinn(II)-chlorid, Eisenpulver, Na₂S und NaHS umfasst;
vorzugsweise wird die Reduktionsreaktion in einem vierten Lösungsmittel durchgeführt, und das vierte Lösungsmittel ist ein gemischtes Lösungsmittel aus Alkohol/Säure, wobei der Alkohol ausgewählt ist aus einem oder mehreren von Methanol, Ethanol und Ethylenglykol, und die Säure ausgewählt ist aus Salzsäure, Essigsäure und wässriger Ammoniumchloridlösung; und
vorzugsweise wird ein Reaktionssystem bei dem Prozess der Reduktionsreaktion zum Rückfluss erhitzt.

8. Herstellungsverfahren für eine freie Base als Zwischenprodukt eines wasserlöslichen Magnololderivats und Honokiolderivats, wobei das Zwischenprodukt in Form einer freien Base eine in Formel III dargestellte Struktur hat: 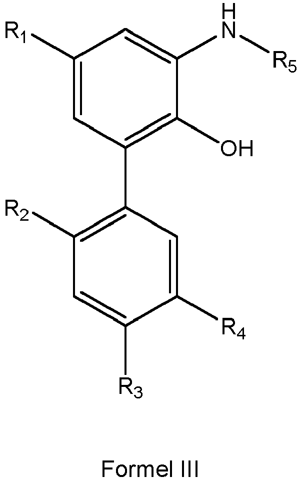
R₁, R₂, R₃ und R₄ in Formel III haben dieselbe Definition wie in einem der Ansprüche 1 bis 5, und R₅ ist ausgewählt aus einem Acylrest, der gebildet wird durch Entfernen eines Hydroxyls eines Carboxyls einer einzelnen Aminosäure oder eines Peptids während einer Kondensationsreaktion des Carboxyls; und
das Herstellungsverfahren umfasst die folgenden Schritte:
Verwenden des Herstellungsverfahrens nach Anspruch 6 oder 7, um das in Formel II dargestellte aminosubstituierte Zwischenprodukt herzustellen;
Durchführen der Kondensationsreaktion zwischen dem aminosubstituierten Zwischenprodukt und einer durch tert-Butoxycarbonyl geschützten einzelnen Aminosäure oder einem durch das tert-Butoxycarbonyl geschützten Peptid, um ein Kondensationsprodukt zu erhalten; und
Umsetzen des Kondensationsprodukts mit Chlorwasserstoff, und dann Alkalisieren mit Ammoniakwasser, Extrahieren und Umkristallisieren, um das Zwischenprodukt in Form einer freien Base zu erhalten.

9. Herstellungsverfahren für eine freie Base als Zwischenprodukt des wasserlöslichen Magnololderivats und Honokiolderivats nach Anspruch 8, wobei die einzelne Aminosäure ausgewählt ist aus einem von Lysin, Methionin, Tryptophan, Valin, Alanin, Phenylalanin, Leucin, Isoleucin, Glycin, Histidin, Arginin, Prolin, Glutamat, Cystin und Asparaginsäure, und das Molekulargewicht des Peptids beträgt ≤ 2500 Da;
vorzugsweise beträgt das Molverhältnis des Zwischenprodukts in Form eines Aminosubstituenten zu der einzelnen, durch tert-Butoxycarbonyl geschützten Aminosäure oder dem durch tert-Butoxycarbonyl geschützten Peptid (1,2~0,8):1;
vorzugsweise wird die Kondensationsreaktion in einem fünften Lösungsmittel durchgeführt, das fünfte Lösungsmittel ist ein nichtreaktives Lösungsmittel, und vorzugsweise ist das fünfte Lösungsmittel ausgewählt aus einem oder mehreren von Dichlormethan, 1,2-Dichlorethan, Ethylacetat, Tetrahydrofuran und N,N-Dimethylformamid;
vorzugsweise beträgt die Reaktionstemperatur bei dem Prozess der Kondensationsreaktion 0~30°C;
vorzugsweise wird der Reaktionsprozess zwischen dem Kondensationsprodukt und Chlorwasserstoff in einem sechsten Lösungsmittel durchgeführt, das sechste Lösungsmittel ist ein nichtreaktives Lösungsmittel, und vorzugsweise ist das sechste Lösungsmittel ausgewählt aus einem oder mehreren von Ether, Ethylacetat, Dichlormethan und 1,4-Dioxan.

10. Herstellungsverfahren für ein wasserlösliches Magnololderivat und Honokiolderivat, wobei das wasserlösliche Magnololderivat und Honokiolderivat eine in Formel IV dargestellte Struktur haben: 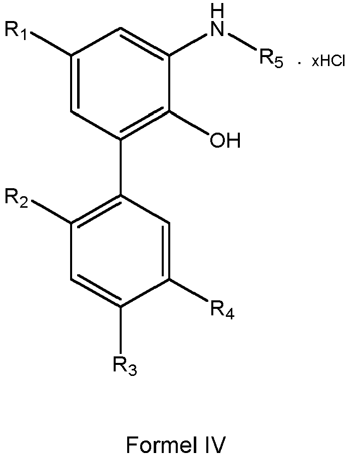 R₁, R₂, R₃, R₄ und R₅ in Formel IV haben dieselbe Definition wie die in Anspruch 8 oder 9; x ist die Zahl der in R₅ enthaltenen versalzten Aminogruppen; und das Herstellungsverfahren umfasst die folgenden Schritte:
Verwenden des Herstellungsverfahrens nach Anspruch 8 oder 9, um das in Formel III dargestellte Zwischenprodukt in Form einer freien Base herzustellen;
Umsetzen des Zwischenprodukts in Form einer freien Base mit Salzsäure, Konzentrieren oder Gefriertrocknen, um das wasserlösliche Magnololderivat und Honokiolderivat zu erhalten.

11. Monohydroxyschutz-Zwischenprodukt eines wasserlöslichen Magnololderivats und Honokiolderivats, wobei das Monohydroxyschutz-Zwischenprodukt eine in der nachstehenden Formel V dargestellte Struktur hat: 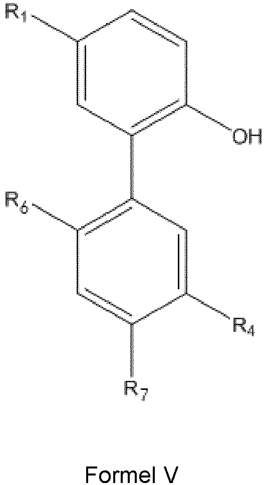 wobei in Formel V R₁ und R₄ dieselbe Definition haben wie oben; R₆ ist 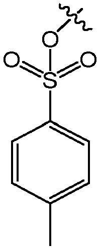 und R₇ ist H; oder R₆ ist H und R₇ ist

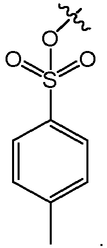

## Revendications

1. Procédé de préparation d'un intermédiaire de nitrification d'un dérivé de magnolol et dérivé d'honokiol hydrosolubles, dans lequel l'intermédiaire de nitrification présente une structure représentée par la formule I : 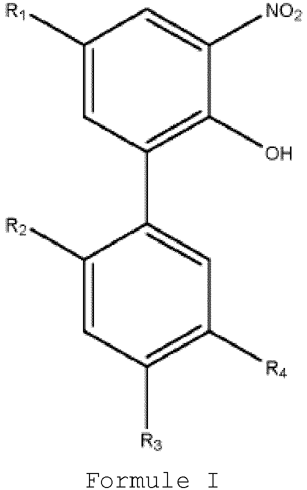
dans la formule I, R₂ est un hydroxyle et R₃ est H ; ou, R₂ est H, et R₃ est un hydroxyle ; et R₁ et R₄ sont sélectionnés indépendamment parmi les groupes donneurs d'électrons en C₁ à C₁₂ ; et
le procédé de préparation comprend les étapes suivantes :
la mise en œuvre d'une protection de monohydroxy sur un composé A 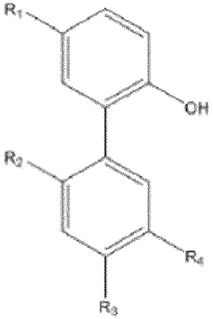 avec un réactif de protection d'hydroxyle en présence d'un agent se liant à un acide pour former un composé à monohydroxy protégé, dans lequel R₁, R₂, R₃ et R₄ dans le composé A ont la même définition que ci-dessus, et les réactifs de protection d'hydroxy sont le chlorure de p-toluènesulfonyle et le 1-hydroxy-benzotriazole ; et
la mise en œuvre d'une réaction de nitrification et d'une réaction de déprotection sur le composé à monohydroxy protégé successivement pour obtenir l'intermédiaire de nitrification.

2. Procédé de préparation de l'intermédiaire de nitrification du dérivé de magnolol et dérivé d'honokiol hydrosolubles selon la revendication 1, dans lequel le groupe donneur d'électrons en C₁ à C₁₂ est sélectionné parmi un alkyle en C₁ à C₁₂ ou un alcényle en C₁ à C₁₂ ;
de préférence, le groupe donneur d'électrons en C₁ à C₁₂ est sélectionné parmi les groupes méthyle, éthyle, n-propyle, isopropyle, n-butyle, isobutyle, tert-butyle, n-pentyle, n-hexyle, vinyle, allyle, octyle, heptyle, propényle, but-1-ényle, but-2-ényle, but-3-ényle, pent-1-ényle, pent-2-ényle, pent-3-ényle, pent-4-ényle, hex-1-ényle, hex-2-ényle, hex-3-ényle, hex-4-ényle, hex-5-ényle, hept-1-ényle, hept-2-ényle, hept-3-ényle, hept-4-ényle, hept-5-ényle, hept-6-ényle, oct-1-ényle, oct-2-ényle, oct-3-ényle, oct-4-ényle, oct-5-ényle, oct-6-ényle ou oct-7-ényle ; et
de préférence, R₁ et R₄ sont identiques.

3. Procédé de préparation de l'intermédiaire de nitrification du dérivé de magnolol et dérivé d'honokiol hydrosolubles selon la revendication 1, dans lequel dans le procédé de la réaction de protection de monohydroxy, le rapport molaire du composé A par rapport au chlorure de p-toluènesulfonyle est de 1: (0,75 à 1), et le rapport molaire du composé A par rapport au 1-hydroxybenzotriazole est de 1: (0,75 à 1) ;
de préférence, la réaction de protection de monohydroxy est réalisée dans un premier solvant, et le premier solvant est un solvant hydrophobe non réactif ;
de préférence, le premier solvant est sélectionné parmi un ou plusieurs parmi le dichlorométhane, le chloroforme, le 1,1-dichloroéthane, le méthyltert-butyléther et le toluène ;
de préférence, le rapport molaire de l'agent se liant à un acide par rapport au réactif de protection d'hydroxy est de (2 à 3):1 ;
de préférence, l'agent se liant à un acide est une base organique ; et de manière davantage préférée, la base organique est sélectionnée parmi un ou plusieurs parmi la pyridine, la 4-diméthylamino-pyridine, le 1,8-diaza-bicycloundécéno-7-ène, la triéthylamine et la N,N-diiso-propyléthylamine ;
de préférence, la température réactionnelle de la réaction de protection de monohydroxy est de -10 °C à 25 °C, de manière davantage préférée de 0 à 10 °C, et le temps de réaction est de 6 à 10 h ; et
de préférence, dans la réaction de protection de monohydroxy, la concentration molaire du composé A est inférieure à 0,2 mol/l par rapport au volume du premier solvant, et de manière davantage préférée, la concentration molaire du composé A est inférieure à 0,1 mol/l.

4. Procédé de préparation de l'intermédiaire de nitrification du dérivé de magnolol et dérivé d'honokiol hydrosolubles selon l'une quelconque des revendications 1 à 3, dans lequel l'étape de la réaction de nitrification comprend : la réaction du composé à monohydroxy protégé avec 60 à 70 % en poids d'acide nitrique pour obtenir un produit de nitrification ;
de préférence, la réaction de nitrification est réalisée dans un deuxième solvant, et le deuxième solvant est un solvant non réactif ;
de préférence, le deuxième solvant est sélectionné parmi un ou plusieurs parmi le dichlorométhane, le 1,2-dichloro-éthane, l'acétate d'éthyle, le méthyltert-butyléther et l'acide acétique ;
de préférence, dans le procédé de la réaction de nitrification, de l'acide nitrique est ajouté au deuxième solvant contenant le composé à monohydroxy protégé selon un mode d'ajout goutte à goutte, et il est mis à réagir à une température de 0 à 25 °C pour obtenir le produit de nitrification ; et
de préférence, calculé avec de l'acide nitrique à 65 % en poids, le rapport pondéral du composé à monohydroxy protégé par rapport à l'acide nitrique est de 3,2 à 4,2:1.

5. Procédé de préparation de l'intermédiaire de nitrification du dérivé de magnolol et dérivé d'honokiol hydrosolubles selon la revendication 1, dans lequel l'étape de la réaction de déprotection comprend :
le mélange du produit de nitrification avec un troisième solvant pour former une solution mélangée, dans lequel le troisième solvant est un solvant non réactif, et de préférence, le troisième solvant est sélectionné parmi un ou plusieurs parmi le 1,4-dioxane, le n-propanol, l'éthylèneglycol et le toluène ; et
l'ajout d'une solution aqueuse d'un hydroxyde de métal alcalin à la solution mélangée et un chauffage, et une réaction pour obtenir l'intermédiaire de nitrification.

6. Procédé de préparation d'un intermédiaire amino-substitué d'un dérivé de magnolol et dérivé d'honokiol hydrosolubles, dans lequel l'intermédiaire amino-substitué présente une structure représentée par la formule II : 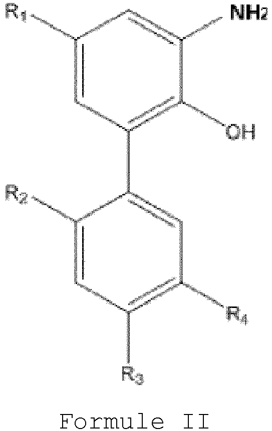
R₁, R₂, R₃ et R₄ dans la formule II ont les mêmes définitions que celles dans l'une quelconque des revendications 1 à 5 ; et
le procédé de préparation comprend les étapes suivantes :
l'utilisation du procédé de préparation selon l'une quelconque des revendications 1 à 5 pour préparer l'intermédiaire de nitrification représenté par la formule I ; et
la mise en oeuvre d'une réaction de réduction sur l'intermédiaire de nitrification pour obtenir l'intermédiaire amino-substitué.

7. Procédé de préparation de l'intermédiaire amino-substitué du dérivé de magnolol et dérivé d'honokiol hydrosolubles selon la revendication 6, dans lequel un réactif réducteur utilisé dans l'étape de la réaction de réduction comprend l'un parmi le chlorure stanneux, une poudre de fer, Na₂S et NaHS ;
de préférence, la réaction de réduction est réalisée dans un quatrième solvant, et le quatrième solvant est un solvant mixte alcool/acide, dans lequel l'alcool est sélectionné parmi un ou plusieurs parmi le méthanol, l'éthanol et l'éthylèneglycol, et l'acide est sélectionné parmi l'acide chlorhydrique, l'acide acétique et une solution aqueuse de chlorure d'ammonium ; et
de préférence, dans le procédé de la réaction de réduction, un système réactionnel est chauffé à reflux.

8. Procédé de préparation d'un intermédiaire de type base libre d'un dérivé de magnolol et dérivé d'honokiol hydrosolubles, dans lequel l'intermédiaire de type base libre présente une structure représentée par la formule III : 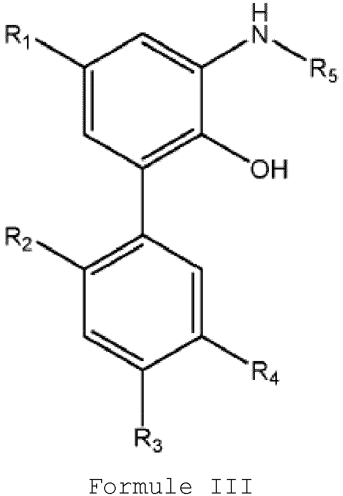
R₁, R₂, R₃ et R₄ dans la formule III ont les mêmes définitions que celles dans l'une quelconque des revendications 1 à 5, et R₅ est sélectionné parmi un résidu acyle formé par l'élimination d'un hydroxyle d'un carboxyle d'un unique acide aminé ou d'un peptide pendant une réaction de condensation du carboxyle ; et
le procédé de préparation comprend les étapes suivantes :
l'utilisation du procédé de préparation selon la revendication 6 ou 7 pour préparer l'intermédiaire amino-substitué représenté par la formule II ;
la mise en oeuvre de la réaction de condensation entre l'intermédiaire amino-substitué et un unique acide aminé protégé par un tert-butoxycarbonyle ou un peptide protégé par un tert-butoxycarbonyle pour obtenir un produit de condensation ; et
la réaction du produit de condensation avec du chlorure d'hydrogène, et ensuite une alcalinisation avec de l'eau ammoniacale, une extraction et une recristallisation pour obtenir l'intermédiaire de type base libre.

9. Procédé de préparation de l'intermédiaire de type base libre du dérivé de magnolol et dérivé d'honokiol hydrosolubles selon la revendication 8, dans lequel l'unique acide aminé est sélectionné parmi la lysine, la méthionine, le tryptophane, la valine, l'alanine, la phénylalanine, la leucine, l'isoleucine, la glycine, l'histidine, l'arginine, la proline, le glutamate, la cystine et l'acide aspartique, et le poids moléculaire du peptide est ≤ 2500 Da ;
de préférence, le rapport molaire de l'intermédiaire amino-substitué par rapport à l'unique acide aminé protégé par un tert-butoxycarbonyle ou au peptide protégé par un tert-butoxycarbonyle est (1,2 à 0,8):1 ;
de préférence, la réaction de condensation est réalisée dans un cinquième solvant, le cinquième solvant est un solvant non réactif, et de préférence le cinquième solvant est sélectionné parmi un ou plusieurs parmi le dichlorométhane, le 1,2-dichloroéthane, l'acétate d'éthyle, le tétrahydrofurane et le N,N-diméthylformamide ;
de préférence, dans le procédé de la réaction de condensation, la température réactionnelle est de 0 à 30 °C ;
de préférence, le procédé de réaction entre le produit de condensation et le chlorure d'hydrogène est réalisé dans un sixième solvant, le sixième solvant est un solvant non réactif, et de préférence le sixième solvant est sélectionné parmi un ou plusieurs parmi l'éther, l'acétate d'éthyle, le dichlorométhane et le 1,4-dioxane.

10. Procédé de préparation d'un dérivé de magnolol et dérivé d'honokiol hydrosolubles, dans lequel le dérivé de magnolol et dérivé d'honokiol hydrosolubles présentent une structure représentée par la formule IV : 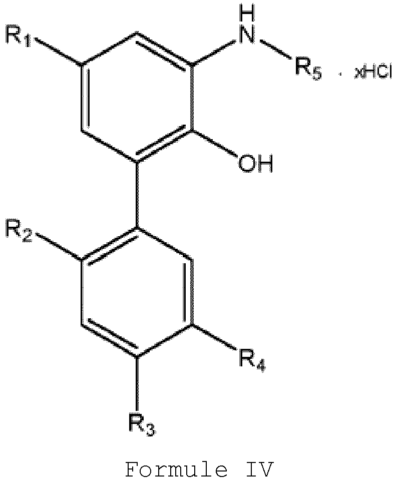 R₁, R₂, R₃, R₄ et R₅ dans la formule IV ont les mêmes définitions que celles dans la revendication 8 ou 9 ; x est le nombre de groupes amino salifiés contenus dans R₅ ; et le procédé de préparation comprend les étapes suivantes : l'utilisation du procédé de préparation selon la revendication 8 ou 9 pour préparer l'intermédiaire de type base libre représenté par la formule III ;
la réaction de l'intermédiaire de type base libre avec de l'acide chlorhydrique, une concentration ou une lyophilisation, pour obtenir le dérivé de magnolol et dérivé d'honokiol hydrosolubles.

11. Intermédiaire de protection de monohydroxy d'un dérivé de magnolol et dérivé d'honokiol hydrosolubles, dans lequel l'intermédiaire de protection de monohydroxy présente une structure représentée par la formule V ci-dessous : 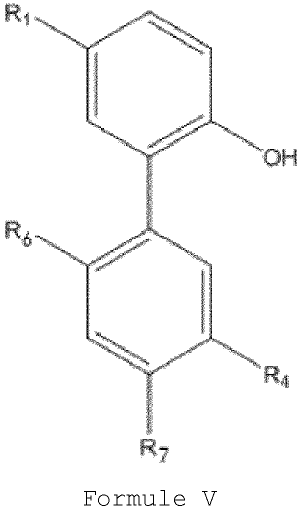 dans lequel, dans la formule V, R₁ et R₄ ont les mêmes définitions que ci-dessus ; R₆ est 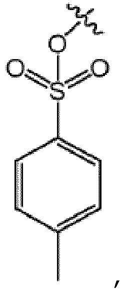 et R₇ est H ; ou, R₆ est H, et R₇ est
